# EUROPEAN PATENT APPLICATION

(11) **EP 3 418 721 A1**
(43) Date of publication of application: **26.12.2018**
(21) Application number: 17177619.8
(22) Date of filing: 23.06.2017
(51) Int. Cl.: G01N 15/14, C12N 5/076

(54) **A MICROFLUIDIC CHIP**

(71) Applicant: Cellix Limited, 12 Dublin (IE)
(72) Inventor: KASHANIN, Dmitry, Dublin, 12 (IE); SHVETS, Igor, Dublin, 12 (IE)
(74) Representative: Purdy, Hugh Barry

(57) **Abstract**

A microfluidic chip for microfluidic flow cytometry analysis of an anisotropic particulate containing fluid, comprises a microfluidic channel having a fluid inlet for receipt of a stream of anisotropic particulate containing fluid, a detection zone comprising at least one pair of detection electrodes in electrical communication with the microfluidic channel configured to generate an AC detection electrical field between the detection electrodes and detect AC impedance changes in the microfluidic channel resulting from anisotropic particles passing between the detection electrodes, and an alignment zone proximal to the detection zone located upstream from the detection zone comprising at least one pair of alignment electrodes in electrical communication with the microfluidic channel configured to generate an alignment electric field between the alignment electrodes.

## Description

### Field of the Invention

The present invention relates to a microfluidic chip for microfluidic flow cytometry analysis of an anisotropic particulate containing fluid. Also contemplated are methods for microfluidic flow cytometry analysis of an anisotropic particulate containing fluid.

### Background to the Invention

Conventional flow cytometry relies on alignment of cells within the sample liquid in a train and detecting, identifying the cells in one-by-one fashion. For example the cells could be aligned in a train that passes through an optical detection beam, so that cells come into the focused beam of the detection apparatus one cell at a time, and the identification is based on optical signal altered by the cell, e.g. scattering of light or fluorescence signal. The same approach is applied in the case of counting and identification of particles in a particle suspending fluid. In this disclosure we will consider both, the fluid containing cells and fluid containing particles, and for brevity shall call them both particulate containing fluid. The focus of attention of this invention is the methods and apparatus for such detection and identification that performs the measurements in a microfluidic format.
Currently in conventional flow cytometry, a stream of particles or cells is positioned within a detection area that sometimes is also called flow cell, by applying a stream of sheath fluid, which surrounds the stream of particulate containing fluid and coaxially focuses the sample stream to achieve uniform flow of cell passing the detector one-by-one. Typically, the flow rate of the sheath stream is one-to-two orders of magnitude greater than of the sample stream to achieve appropriate focusing ratio and obtain the sample stream of 30-70 micrometres in diameter that is a suitable size for the optical-detection-based cytometers. In the fluorescence scattering flow cytometry, flowing cells or particles pass through a focused laser beam inside the detection channel and scattering of laser light is measured. Two directions of light scattering are detected independently: forward light scattering, which contains information about the particles size and side light scattering, which contains information about internal properties of the cell or particles. Additionally, side light scattering signal might be subject to light filtering in order to extract information about certain fluorescence emission bands of the particle or cell. It is important to note that hydrodynamic focusing is necessary to reduce variation of scattered light signal by making sure that all particles or cells are subjected to the same intensity of laser light and pass through the focused laser beam uniformly. Uniformity implies uniformity of the trace of the cells or particles in the focused laser beam as this affects the intensity of the detected signal. If the particles or cells are not of circular shape as they often are, uniformity also refers to of their orientation with respect to the focused beam direction. Indeed if the cells are e.g. discoid, then the optical signal will change depending on whether they face the optical beam facing with larger area side (flat face) or smaller area side (edge of the discoid). Particles or cells then can be divided into subpopulations based on their respective fluorescence intensities in a certain fluorescence band. These subpopulations could correspond to respective staining of particular cell receptors, cytoplasm or nucleus. It is becoming more important to differentiate particles or cells, which differ by minute change of particular cell properties. For example, accurate measurement of cell size allows detecting abnormal red blood cells amongst healthy red blood cells. Ability to differentiate cell shape allows detection of sickle cells and for differentiation of different types of bacteria, for example rod shaped E-coli or circular shaped Staphylococci bacteria. Measurement of content of the cell nucleus allows for example for differentiation of X and Y bearing chromosomes in spermatozoa cells. Such measurements of minute difference of these cell properties are beyond current advancements in conventional flow cytometry. The main limitation preventing more accurate measurements is inability of current techniques to present all the cells or particles in the particulate fluid in front of the detector in a reproducible and accurate manner.

In recent years there is increasing interest in implementing cell flow cytometry devices in a microfluidic format. Microfluidics provides a convenient technology platform to miniaturize conventional scattering flow cytometry making construction and manufacturing of conventional flow cytometry detection channel simple, miniature while also making the whole device disposable.

There are several examples of microfluidic flow cytometers utilizing fluorescence and impedance detection on chip in order to count the cells and to evaluate cell properties. Article "Microflow cytometer with integrated hydrodynamic focusing", Marcin Frankowski et. al. ["Microflow cytometer with integrated hydrodynamic focusing", Marcin Frankowski et. al., Sensors 2013, 13, 4674-4693] describes several configurations of integrated cytometer on microfluidic chip including hydrodynamic focusing and also describes experiments with fluorescence detection of calibration beads with various fluorescent intensities. The method provided allows for low CV of measurements for intensity of particles around 3%, which is comparable with conventional flow cytometry measurements. Moreover authors have experimented with detection of fluorescently tagged lymphocyte subpopulation, with results comparable with those of conventional cytometry.

The publication "Microfluidic impedance cytometer for platelet analysis", Mikael Evander et.al ["Microfluidic impedance cytometer for platelet analysis", Mikael Evander et.al, Lab on a chip, volume 13, 2013] describes impedance-based flow cytometer including two dimensional hydrodynamic focusing with dielectric sheath for the detection of platelets among red blood cells. To calibrate the system, authors used 10 micrometer and 5 micrometer polystyrene beads and measured the impedance signal produced by the beads at various ratios of sample and sheath fluids. As a result they have achieved best signal and lowest variation by using dielectric sheath and at the core sample stream width of 33 micrometers, versus initially used 145 micrometers. The authors have also experimented with TRAP activated platelets versus non-activated platelets from healthy donors and were able to detect the differences between the two populations.

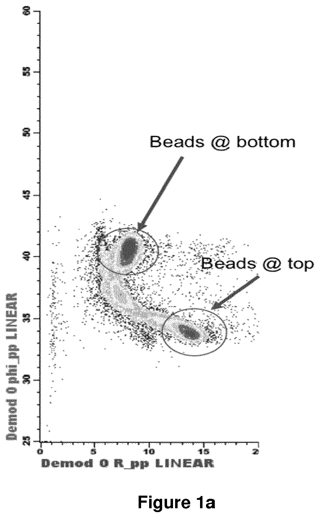

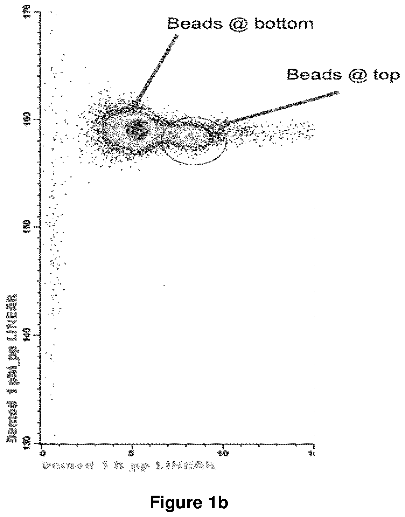

The inventors have also investigated impedance flow spectroscopy method of cell detection where two pairs of electrodes are used similar to configuration described in ["Microfluidic impedance cytometer for platelet analysis", Mikael Evander et.al, Lab on a chip, volume 13, 2013], each pair having an excitation and measurement electrodes. An AC voltage at radio frequency from 100 KHz -100 MHz is applied to an excitation electrodes and an electrical current is measured by the measurement electrodes. The electrical current being measured is then amplified and converted into an output voltage. The output signal is then demodulated to remove excitation frequency and to recover impedance magnitude and phase. As a cell passes through the pair of excitation and measurement electrodes, impedance magnitude and phase change, thus recording the information about the cell properties. Additional pair of electrodes ensures measurement is differential thus eliminating parasitic electromagnetic noise.
Typically, the measurement is taken at low frequency: 200KHZ-500KHZ to acquire information about the cell size and at high frequency 2-50MHz to acquire information about the cytoplasm and internal properties of the cell. The Figure 1a and Figure 1b display density plot of impedance magnitude versus phase for a population of identical polystyrene beads at low frequency 0.5MHz (Figure 1 a) and high frequency 10 MHz (Figure 1 b). The beads are polystyrene beads of 6 micrometer in diameter. In that experiment the channel is of a square cross-section (30 micrometers x 30 micrometers) and there are two electrodes deposited on the channel with the size of 20 micrometers x 0.2 micrometers: the top electrode deposited on the upper wall of the channel (ceiling of the channel) is the excitation electrode and the electrode at the lower wall of the channel (floor of the channel) is the signal electrode. When particles pass in between the excitation and the signal electrode, they induce a significant variation in complex value of impedance comprised of variations in the magnitude of the impedance and also the variation in the phase of the impedance, equivalent to variation in real and imaginary parts of impedance. At low frequency, the impedance signal depends on the cell size and also on the cell position within the microfluidic channel. Moreover, it demonstrates that signal is different for the cells flowing at the top of the channel and close to excitation electrode versus those flowing at the bottom of the channel and close to signal electrode. The difference is partly due to non-uniformity of the electric field between two electrodes of a finite width. Additionally, there are differences of electric field gradient at the top and at the bottom of the channel; especially, the electric field gradient is greater at the excitation electrode compared to the one at the signal electrode. This also contributes to the sensitivity of impedance signal to the cell position at high frequencies. In this document we shall refer to this format of data presentation as impedance density plot, similar to the way it is referred to in Figures 1 a and 1 b. It is important to note that in case of polystyrene beads and also cells measurements, we have not employed any hydrodynamic focusing or positioning of the sample fluid within the microfluidic channel, and yet achieved separation of homogeneous population into several distinct subpopulations. Further we give explanation of this phenomenon and will provide the method to utilize it to our advantage.

It is known from the prior art publications that there are situations when distribution of particles flowing across the microchannel of rectangular cross-section is inherently non-uniform. Moreover, depending on particle velocity and size, the particles arrange into preferred stable positions, which are typically not in the center of the rectangular microchannel. These hydrodynamically favoured positions might be influenced by ratio of width and the height of the channel (square channels versus rectangular channels), and also by the velocity and the size of the particles, viscosity of fluid and by the Reynolds number and the densities of the particles and the liquid moving the particles. The self-focusing of particles is often referred to as an inertial particle focusing which occurs at the flows with Reynolds number higher than unity (Stokes flow) and lower than one hundred. This ordering occurs due to four lateral forces acting on the particle flowing in the rectangular walled channel: Magnus force due to slip-rotation, Saffman force due to slip-shear, wall lift force due to the disturbance of flow field around particles from wall, and shear gradient lift force due to the parabolic curvature of the undisturbed velocity profile ["Inertial microfluidic physics", Hamed Amini et. al., Lab on a chip, Issue 15, 2014]. Publication "Fundamentals and Applications of Inertial Microfluidics: A Review" Jung Zhang et. al. ["Fundamentals and Applications of Inertial Microfluidics: A Review", Jun Zhang et. al, Lab on a chip, November 2015] provides a review of advances in inertial focusing and summarizes situations where stable positions are influenced by geometry of the channels. According to the article, if flown in a circular channel, randomly distributed particles migrate towards stable positions, which are located equidistantly and 0.6 times of the channel radius from the circular channel axis. In a square straight channel, where width is equal to depth of the channel, particles focus normally in four equilibrium positions facing the center of each wall. If the channels are rectangular and the aspect ratio is less than 0.5 (the width is at least 2 times higher than the depth) there are only two stable positions at the centers of longer walls. This phenomenon is explained by Jian Zhou et. al. ["Fundamentals of inertial focusing in microchannels", Jian Zhou et.al., Lab on a chip, Issue 6, 2013] and it is due to a two-stage inertial focusing. Moreover, techniques described in Dino Di Carlo et. al. ["Continuous inertial focusing, ordering, and separation of particles in microchannels", Dino Di Carlo et.al, PNAS, volume 104, No 48, November 2007] allow for the focusing of particles in the stable positions and simultaneously for the rotation of non-circular particles, where rotational alignment is observed with the disk of discoid particles parallel to the wall of the channel.

Additionally to the inertial focusing, there are number of hydrodynamic sample focusing techniques implemented on a chip where microfluidic channels are added to the detection channel in order to position sample stream within the detection channel. These additional channels carry sheath fluid, which is similarly to conventional flow cytometry, envelops the sample carrier fluid. There are a number of 2D focusing techniques focusing in a single plane. More recently, 3D focusing techniques emerged with additional focusing perpendicular to the plane. These are described and referenced below.

In recent years, microfluidic impedance cytometry has been further developed to count and discriminate between different kinds of cells. Multi-frequency impedance measurements can be used to determine the electrical properties of single cells in a microchip [S. Gawad, L. Schild, P.H. Renaud, Micromachined impedance spectroscopy flow cytometer for cell analysis and particle sizing, Lab. Chip. 2001 1 76-82; T.Sun and H. Morgan, Single-cell microfluidic impedance cytometry: a review, Microfluid. Nanofluid, 2010, 8, 423-443]. In these methods, cells flow between miniature electrodes which have an AC field applied across them. As the cell passes between the electrodes, the current path is disturbed and the change in current gives a change in the impedance signal associated with a single cell. Usually, impedance measurements at the frequency of (1-5 MHz) give information on the cell membrane capacitance whilst much higher frequencies (>10 MHz) probe the internal properties of the cell. Two or more frequencies can be applied simultaneously to differentiate different types of cells. Impedance flow cytometry can readily detect a cell, and the original technique was developed by Coulter for this. When it comes to more challenging task of separating the sub-populations of cells within the sample fluid, the performance of the impedance cytometry is much less convincing due to large spread in the data points corresponding to each cell. Integration of 3D hydrodynamic focusing with a conventional type microfluidic chip is also not simple. The performance of such on-chip 3D focusing has limited capability.
To reduce the CV of the impedance cytometry it is desirable to be able to direct the sample flow through a well-defined point in between the electrodes, e.g. the centre of the channel. This reduces the spread in the data points from a single population of cells of type of particles in the flow. It may also be desirable to align all the cells (particles) in the same way with respect to the direction of the electric field created by the electrodes. Cells often do not have an overall spherical shape but are rather elongated, ellipsoidal or discoid in shape. The signal from the cell in electrical impedance cytometry device depends on the orientation of the elongated axis of the cell with respect to the electrodes.

The technique called hydrodynamic focusing has been used to direct the flow of particles or cells through the centre of the channel and it has been known for decades. The phenomenon was described as early as in the year 1883 [O.Reynolds, Proc. R. Soc. London, 1883, 35 84-99] and it was originally related to the confinement of the sample flow flanked on both sides by sheath flow streams. The cross-section of the sample liquid flow in a flow cytometer is typically in the range of 0.003-0.03 mm².

Hydrodynamic focusing is particularly important for the detection of cells and particles on a chip utilizing impedance measurements. Indeed, for identification of cells (or particles) it is necessary to arrange these in such a flow that they pass in front of the detection system one by one. This "one cell-by-one cell" principle is fundamental for the successful cell identification: one needs to avoid the situation of multiple cells passing through the detection system at once as it could prevent the identification. Making the channel so small that cells (particles) align there one by one due to the tight cross-section of the channel, is not practical: such a small channel that is comparable in cross-section with a single cell, is prone to blockage and it would also require a significant pressure difference as the friction of the laminar flow against the walls increases with decreasing channel cross-section. Therefore, it is common to use hydrodynamic focusing. Hydrodynamic focusing is based on injection of the sample fluid into the laminar flow of sheath fluid. The two flows then merge into to a single channel, usually of a reduced cross-section. This reduces the cross-sections of both, the sheath fluid part of the flow and also the sample liquid flow, and thus achieves the desired reduction in the cross-section of the sample fluid flow. To control the cross-section of the sample fluid, one could change the flow rates of the sample fluid and sheath fluid. For example, the flow rate of the sheath fluid could be increased to reduce the cross-section of the sample fluid. Such a small cross-section of the sample fluid flanked by the flow of the sheath fluid passes through a channel of a rather large cross-section, i.e. multiple of the cell size, that does not block. One could say that microfluidic focusing replaces the hard walls of microfluidic channel for fluid quasi-walls and this reduces the risk of the microchannel blocking. In relation to the electrical impedance based cytometry, hydrodynamic focussing reduces the width of the conductive sample stream to the appropriate size of the cells, increasing the percentage resistance change in the conductive path when a cell passes by.

In recent years there is increasing body of work on the use of hydrodynamic focusing in microfluidic chips and microchannels. For example, the Japanese patent laid-open No 2003-107099 discloses a "fractionation microchip having a channel for introducing a particulate-containing solution, and a sheath flow forming channel arranged on at least one lateral side of the of the introducing channel. The fractionation microchip further has "a particulate measuring section for measuring the particulates introduced, at least two particulate fractionating channels disposed on the downstream side of the particulate measuring section so as to perform fractional collection of the particulates, and at least two electrodes disposed in the vicinity of channel ports opening ... so as to control the moving direction of the particulates." The particulate fractionation microchip disclosed in Patent 2003-107099, is so designed that fluid laminar flows are formed by a "trifurcated channel" having a channel for introducing a particulate-containing solution and two sheath flow-forming channels. In essence this is a 2D hydrodynamic focussing on a chip. In the particulate fractionation microchip disclosed in Patent 2003-107099, the trifurcated channel ensures that the particulate-containing solution is sandwiched by the flows of the sheath liquid from the left and right sides, and the particulates are made to flow through the center of the channel in the particulate measuring section. As a result, in the case of measuring the particulates optically, for example, each of the particulates can be accurately irradiated with measuring light. Similar approach is described in [R. Rodriguez-Trujillo, C. Mills, J. Samitier, G. Gomila, Microfluid. Nanofluid, 3 171 (2007)] and [P.Walsh, E. Walsh, M. Davies, Int. J. Heat Fluid Flow 28 44 (2007)].

The 2D hydrodynamic focussing has its intrinsic limitations. With this in mind, there is an increased effort to introduce a 3D hydrodynamic focussing on a microfluidic chip to confine the sample in both, the horizontal and vertical directions. One solution for integration of such 3D focussing with a conventional type microfluidic chip is described in ["Three-dimensional hydrodynamic focussing in a microfluidic Coulter counter", R. Scott, P. Sethu, C.K. Harnett, Rev. Sci. Instruments 79 046104 (2008)]. The focussing is achieved in using a two level design, the sheath fluid enters the microfluidic chip from a channel that is both, wider and taller than the sample stream.

A similar approach is described in ["Universally applicable three-dimensional hydrodynamic microfluidic flow focussing" Yu-Jui Chiu, S.H. Cho, Z. Mei, V. Lien, T.F. Wu, Y.H. Lo, Lab Chip 2013 13 1803]. That study deals with three-dimensional hydrodynamic focusing where the sample channel and the two sheath channels having a greater height than the sample channel, join at the junction before the main channel which has the same height as the sheath channel. The merging of channels of different heights produces flow confinement both in the lateral and transverse directions, resulting in 3D focused flow. In that publication, 3D focussing refers to the confinement of sample flow to a straight line at the centre of a channel of a conventional microfluidic planar chip. The authors of that publication state that "particles have a tendency to settle in positions away from the centre of the channel. The flow focussing needs to counter such effects". Therefore, the trend in the microfluidic devices is to position particles at the centre of the microfluidic channel.

In this document we will extensively refer to dielectrophoretic torque. This should not be confused with the dielectrophoretic force that has been used for separation of cells. We shall therefore explain the terms dielectrophoresis and dielectrophoretic force. In contrast to electrophoresis, where cells move in a uniform electric field due to their surface charge, dielectrophoresis refers to the movement of particles (cells) in a non-uniform electric field due to their polarizability. For movement in response to a dielectrophoretic force, particles (cells) do not need to possess a surface charge. The surface charges are produced by the field by disproportionation (pulling) the opposite charges to different parts of the cell. The disproportionation could result from the very nature of the cell itself, its interaction with the surrounding fluid or its interaction with the AC electric field. Once exposed to a gradient field, cells migrate either toward or away from the region of strongest field intensity depending on the electrical permeability (permittivity) of the cell and the fluid. Cells with a higher permeability than the fluid are attracted toward the field maxima, which is known as positive dielectrophoresis. The opposite is true for negative dielectrophoresis. The magnitude of the dielectrophoretic force is dependent on the size and properties of the cell, fluid, and the parameters of the electric field, which is useful for sorting cells by size and dielectric properties. We stress that the dielectrophoresis is based on gradient-, i.e. non uniform field. Usually papers described using AC gradient field, i.e. AC field with amplitude that varies strongly in space as DC field may be screened by a conducting liquid surrounding the cells or by the cells themselves.

Dielectrophoresis force was used by a number of authors for the separation of cells and other microscopic objects. The review [M.P. Hughes, Strategies for dielectrophoretic separation in laboratory on chip format, Electrophoresis 2002, 23, 2569-2582] describes strategies for the separation of bioparticles such as cells, viruses and proteins in miniaturised laboratory-on-a-chip format. The separation principle in based on difference in polarisation of particles. The review describes various electrodes such as stepped electrodes, "Christmas tree" electrodes, spiral electrodes creating controlled gradient (non-uniform) electric field. Publication [B.H. Lapizco-Encinas, B.A. Simmons, E.B. Cummings, Y. Fintchenko, Dielectrophoretic concentration and separation of live and dead bacteria in an array of insulators, Anal Chem 2004, 76, 1571-1579]. The paper [N. Lewpiriyawong, K. Kandaswamy, C. Yang, V. Ivanov, R. Stocker, Mcrofluidic characterization and continuous separation of cells and particles using conducting poly(dimethyl siloxane) electrode induced alternating current-dielectrophoresis, Anal. Chem. 2011, 83, 9579-9585] describes separation of live yeast cells from the dead ones in a microfluidic channel under the influence of the dielectrophoretic forces in gradient electric field along the channel. The principle is based on the different value of the dielectrophoretic force acting on live and dead cells. A similar method is described in the paper [N.Lewpiriyawong, C Yang, Y.C. Lam, Continuous sorting and separation of microparticles by size using AC dielectrophoresiss in a PDMS microfluidic device with 3-D conducting PDMS composite electrodes, Electrophoresis 2010, 31, 2622-2631]. Other publications [H. Shafiee, M.B. Sano, E.A. Henslee, J.L. Caldwell, R.V. Davalos, Selective isolation of live/dead cells using contactless dielectrophoresis (cDEP), Lab Chip, 2010, 10, 438-445] and [H. Li, R. Bashir Dielectrophoretic separation and manipulation of live and heat treated cell of Listeria on microfabricated devices with integrated electrodes, Sensors and Actuators B 86 (2002) 215-221] describe a similar approach.

In this document we shall deal with the rotation of cells (particles) by a field having stationary vectorial direction. This should not be confused with electrorotation. In electrorotation the cells are subjected to a rapidly rotating quadrupole field, typically rotating in space with the frequency of 10⁴-10⁷ revolutions per second. Ability of the cell overall, or elements of the cell to participate in rotational movement contains signature of the cell type, status and condition. The signature is contained in frequency (spectral) dependencies and amplitude of the rotating field. One of first papers on the subject is [W.M. Arnold, U. Zimmermann, Electro-rotation: development of a technique for electric measurements of individual cells and particles, Journal of Electrostatics 21 (1988) 151-191]. The paper [A.V. Sokirko, The electrorotation of axisymmetrical cell, Biol. Mem. Vol. 6(4) 1992, 587-600] gives theoretical analysis of the technique. The paper [C. Dalton, A.D. Goater, J.P.H. Burt, H.V. Smith, Analysis of parasites by electrorotation, Journal of Applied Microbiology, 2004, 96, 24-32] describes how the electrorotation could be used to probe physiological structure of micro-organisms to distinguish between different life cycle stages of certain parasites. The paper [C. Dalton, S. Adamia, L.M. Pilarski, K.V.I.S. Kaler, Investigation of human malignant cells by electrorotation, 2004 Annual Report of the Conference "Electrical Insulation and Dielectric Phenomena"] demonstrates separation of lymphocytes into large and small lymphocytes.

To sum up, the importance of positioning of particles (cells) within the channel of a microfluidic flow cytometer chip utilising optical, fluorescence, magnetic or other forms of signal detection is well appreciated. The particles need to be positioned in a train so that they pass the detection area in a one particle (cell) at a time format. Positioning of particles (cells) is even more important for the impedance spectrometer-based analysers. The particles (cells) need all to pass at the same distance away from the electrodes in order to increase the resolution capability of the spectrometer, i.e. its ability to distinguish between different particles (cells).
At present the most common approach to achieving such a positioning is in using hydrodynamic focusing that aligns the particles (cells) along the centre of the microfluidic channel. This is done by means of the guidance (sheath) fluid that envelopes the sample fluid all around and guides the sample fluid through the centre of the microfluidic channel. From the review of current state of the art it is evident that prior art solutions teach extensively how to position the cells within a common microfluidic channel using hydrodynamic focussing, inertial focussing in the flow and focussing into lines of hydrodynamically stable positions traversing through channel. Furthermore this allows aligning cells into a chain so that they pass the detection area in one-by-one file. This allows passing the particles (cells) along similar locations of the detection area, e.g. at similar distance from the detection electrodes and significantly reduces the spread of data points from a set of identical particles or a population of cells making their identification more feasible.
However, the issue arises with anisotropic particles (cells). The prior art, does not completely solve the problem of variation of data points from individual particles (cells) due to the particles (cells) orientation within microfluidic channels. It is clear that it would be beneficial to have additional means for aligning the set of anisotropic particles (cells) all along a single direction. A tighter alignment of anisotropic particles (cells) with reduced angular spread of their orientations would further reduce the scatter of data points and make their identification and subsequent separation, more robust. Furthermore, it would be advantageous to have means of control over the alignment direction with respect to the elements of the detection system. For example, it could be advantageous to align short axis of discoid cell parallel to the detection electrodes. This would allow increasing the signal arising from single particles (cells) thus making their identification and subsequent separation, more robust and detect subpopulations of particles with minute difference from each other. It could also be beneficial to have means for aligning of the particles (cells) that are already pre-aligned by means of hydrodynamic focussing in order to enhance the effect of the hydrodynamic focussing.
Alignment of anisotropic particles is a major issue for microfluidic flow cytometers or particle analysers that is not restricted to AC impedance detection and identification of the cells. Alignment of particles (cells) in a microfluidic channel of a chip could maximize the resolution capability of the cytometer (particle analyser), the cells (particles) that uses other means of particle (cell) detection. here

It is an object of the invention to overcome at least one of the above-referenced problems.

### Summary of the Invention

According to a first aspect of the present invention, there is provided a microfluidic chip for microfluidic flow cytometry analysis of an anisotropic particulate containing fluid, the microfluidic chip comprising:
a microfluidic channel having a fluid inlet for receipt of a stream of anisotropic particulate containing fluid;
a detection zone comprising a sensor configured to detect a change in the microfluidic channel resulting from anisotropic particles passing the sensor (the sensor preferably comprises at least one pair of detection electrodes in electrical communication with the microfluidic channel configured to generate an AC detection electrical field between the detection electrodes and detect AC impedance changes in the microfluidic channel resulting from anisotropic particles passing between the detection electrodes); and an alignment zone proximal to the detection zone located upstream from the detection zone comprising at least one pair of alignment electrodes in electrical communication with the microfluidic channel energised by a voltage/current generator and configured to generate an alignment electric field between the alignment electrodes sufficiently strong to align the anisotropic particulates to produce uniform alignment of anisotropic particulates on their entry to the detection zone.

In one embodiment, the alignment electrical field is a uniform electrical field having substantially no electrical gradient.

In one embodiment, the pair of detection electrodes are configured to generate the detection electrical field extending substantially along a Z-axis of the chip.

In one embodiment, the at least one pair of alignment electrodes is configured to generate the alignment electrical field across the microfluidic channel at an angle of 5-90° to the detection electrical field.

In one embodiment, the pair of alignment electrodes is configured to generate the alignment electrical field across the microfluidic channel at an angle of 30-45° to the detection electrical field.

In one embodiment, the pair of alignment electrodes is configured to generate the alignment electrical field across the microfluidic channel at an angle of 40-45° to the detection electrical field.

In one embodiment, the pair of alignment electrodes comprises an upper electrode disposed above and towards one side of the microfluidic channel and a lower electrode disposed below and towards an opposite side of the microfluidic channel.

In one embodiment, the alignment electrodes extend longitudinally along the microfluidic channel and is arranged such that a degree of overlap between the alignment electrodes and the microfluidic channel varies longitudinally along the microfluidic channel. In one embodiment, the degree of overlap increases along the fluid flow direction. In one embodiment, the degree of overlap decreases along the fluid flow direction.

In one embodiment, the at least one pair of alignment electrodes is configured to align the anisotropic particulates at a first aligned position, and then rotate the anisotropic particulates from the first aligned position to a second aligned position.

In one embodiment, the alignment zone comprises one pair of alignment electrodes configured to align the anisotropic particulates at a first aligned position and a second pair of alignment electrodes distal of the first pair of alignment electrodes configured to rotate the anisotropic particulates to the second aligned position

In one embodiment, the detection pair of electrodes comprise an excitation electrode energised by AC voltage/current generator and a signal electrode coupled to a lock-in amplifier or AC phase sensitive detector.

In one embodiment, the microfluidic channel has a polygonal cross-section.

In one embodiment, the microfluidic chip according to the invention includes hydrodynamic focussing means comprising a guidance microfluidic channel and a sample microfluidic channel that merge proximally of the alignment zone to provide the microfluidic channel.

In one embodiment, the distance from the location of merging of the sample microfluidic channels and the guidance channels is in the range of 100 µm to 50 mm.

In one embodiment, the cross-section of the microfluidic channel is not rectangular.

In one embodiment, the cross-section of the sample channel and/or the guidance channel is not rectangular.

In one embodiment, the cross section of the common channel has dimensions in the range of 10 to 1000 µm and more preferably 20 to 500 µm.

In one embodiment, the microfluidic chip includes a pair of shielding electrodes disposed between the alignment electrode pair and detection electrode pair.

In one embodiment, the direction of the alignment electrical field/fields is chosen such as to be aligned preferably parallel or perpendicular with the anisotropic direction of the particulates resulting from the hydrodynamic focussing defined by the shape of the sample channel, guidance channel, the microfluidic channel and the configuration in which the sample channel and the guidance channel merge to form the microfluidic channel and the anisotropic shape of the particulates.

In one embodiment, the direction of the alignment electrical field is chosen such as to reduce the spread in angular alignment of the particulates present at the point of entry into the alignment zone.

In one embodiment, the at least one pair of alignment electrodes are configured to generate an AC electric field having a frequency such that complex dielectric permittivity of the particulates is significantly different from the one of the fluid carrying these particles.

In one embodiment, the width of the cross section of the microfluidic channel is in the range of 10-500 µm.

In one embodiment, the alignment electrodes are made of one or several layers of metals.

In one embodiment, the alignment electrodes have length along the common channel in the range of 20 µm to 20 mm.

In one embodiment, the alignment electrode/electrodes positioned on each face of the common microfluidic channel are segmented into subsections.

In one embodiment, the alignment electrode/electrodes are positioned along the wall of the microfluidic channel over a mechanical contact area and yet are separated from the interior of the channel for a fraction of the mechanical contact area by a layer of dielectric and make electric contact with the interior of the channel over a fraction of the mechanical contact area via one or several windows formed in the layer of dielectric.

In one embodiment, one or more alignment electrodes are electrically insulated from the interior of the common channel.

In another aspect, the invention provides an apparatus comprising:
a microfluidic chip according to any embodiment described in the summary of invention;
at least one voltage and/or current generator electrically coupled to the pair of alignment electrodes; and
optionally at least one voltage and/or current generator electrically coupled to the pair of detection electrodes.

In one embodiment, the at least one voltage and/or current generator electrically coupled to the pair of alignment electrodes is configured to generate an AC alignment electrical field having a frequency in the range of 10 KHz to 100 MHz.

In one embodiment, the at least one voltage and/or current generator electrically coupled to the pair of alignment electrodes is configured to generate an AC alignment electrical field having a frequency in the range of 50 KHz to 10 MHz.

In one embodiment, the at least one voltage and/or current generator electrically coupled to the pair of alignment electrodes is configured to generate a DC voltage having the value in the range of 0.1-100 V.

In one embodiment, the at least one voltage and/or current generator electrically coupled to the pair of alignment electrodes is configured to generate a DC voltage having the value in the range of 0.5-20 V.

In one embodiment, the microfluidic chip comprises at least two pairs of alignment electrodes, each energised by a generator operating at different frequencies or one of the generators operates as DC voltage and the other one at AC voltage.

In another aspect, the invention provides a method of microfluidic flow cytometry analysis of an anisotropic particulate containing fluid, the method comprising the steps of:
pumping an anisotropic particulate containing fluid through a microfluidic channel of a microfluidic chip or apparatus according to the invention
energising the at least one pair of alignment electrodes to align the particulates in the fluid in the alignment zone;
and detecting impedance changes in the detection zone caused by aligned particulates passing through the detection electrical field.

In one embodiment, the method includes an initial step of hydrodynamically focussing the anisotropic containing fluid before it passes into the alignment zone.

In one embodiment, the at least one pair of alignment electrodes is configured to rotate the particulates into a first common alignment and then further rotate the particulates into a second common alignment.

In one embodiment, the method includes a step of energising the pair of alignment electrodes to produce an AC alignment electrical field having a frequency in the range of 10 KHz to 100 MHz.

In one embodiment, the method includes a step of energising the pair of alignment electrodes to produce an AC alignment electrical field having a frequency in the range of 50 KHz to 10 MHz.

In one embodiment, the method includes a step of energising the pair of alignment electrodes to generate a DC voltage having the value in the range of 0.1-100 V or AC voltage having amplitude in this range.

In one embodiment, the method includes a step of energising the pair of alignment electrodes to generate a DC voltage having the value in the range of 0.5-20 V or AC voltage having amplitude in this range.

In one embodiment, the anisotropic particulates are cells, and in which the method is a method of separating cells according to phenotype

In one embodiment, the anisotropic particulates are sperm cells, and in which the method is a method of separating sperm cells according to sex.

The invention deals with the apparatus and the method for detection of cells or particles based on measurements of complex AC impedance between electrodes across the flow of fluid containing such cells or particles and AC impedance spectroscopy. The electrodes are positioned directly on the walls of the common microfluidic channel, typically along two opposite sides of the channel in direct electric contact with the fluid inside the channel. The detection is done by energising one or more detection excitation electrodes and detection of AC complex signal comprising the amplitude and the phase of the signal from detection electrodes using phase-sensitive detector that is also sometimes referred to as lock-in amplifier. The cells are arranged in between the electrodes of the detection area in a one-by-one file. The signal is altered by each passing cell or particle, and the identification of the cells or particles is done by amplitude and phase characteristics of the signal measured by the lock-in amplifier and their dependencies on the frequency of the AC signal energising the detection electrodes. In order to reduce the statistical spread of data measured from a set of cells or particles by impedance spectroscopy, they need to be consistently and reproducibly positioned in the common microfluidic channel with respect to the detection electrodes as the signal detected depends on the position of the particles with respect to the detection electrodes. Such consistent and controlled positioning of the particles within the common microfluidic channel can be achieved by means of hydrodynamic focussing.

This invention teaches that there is another important factor that defines the statistical spread of data points from the population of cells or particles. The orientation of a cell or particle with respect to the detection electrodes is important in the case of particles (cells) that are not circular in shape, e.g. elongated, elliptical or discotic. We shall call such particles anisotropic in contrast to sphere-like cells or particles. We explain that even a particle (cell) of a sphere-like overall shape can still be anisotropic if its interior AC electric characteristics are direction-dependant. The orientation of the particle (cell) defines the change in AC impedance induced between the electrodes from such single passing anisotropic particles (cells). The invention teaches the device and method that enables achieving uniform alignment of particles (cells) with respect to the detection electrodes. The alignment is based on dielectrophoretic torque acting on the particles. The dielectrophoretic torque is generated by means of AC or DC electric field applied to the particles (cells).The field is generated by energising alignment electrodes positioned proximal the walls of the common microfluidic channel. In the preferred embodiments the alignment electrodes are metal electrodes positioned on opposite sides of the microfluidic channel in direct electric contact with the fluid carrying the particles. These are energised by means of a DC or an AC voltage- or current generator. It should be stressed that the invention is not seeking to utilise electrophoretic or dielectrophoretic force. In fact in the preferred embodiments of the invention there is preferably no electrophoretic or dielectrophoretic force. Dielectrophoretic force is cancelled as the cells are placed at the location of a uniform AC or DC electric field rather than gradient field that is required for generation of dielectrophoretic force. The invention also teaches the method of correct selecting of the AC frequency applied to the alignment electrodes. This is based on achieving a significant difference between the AC complex permittivity of the particles (cells) and that of the fluid carrying the flow of the particles (cells) through the common microfluidic channel.

According to the invention, the alignment electrodes are positioned upstream from the detection area of the microfluidic channel. The distance between the alignment electrodes (alignment area) and the detection area is short enough so that the particles (cells) aligned in the alignment area still arrive aligned into the detection area as they travel downstream from the alignment area into the detection area. We describe an embodiment of this invention where alignment of the particles (cells) is done in conjunction with their identification using AC impedance spectroscopy. Other methods of particles (cells) identification, e.g. fluorescence detection or optical scattering, can also be used in conjunction with the alignment of particles (cells) taught by the invention.

Other aspects and preferred embodiments of the invention are defined and described in the other claims set out below.

### Brief Description of the Figures

Figure 1 a Density plot of impedance magnitude versus impedance phase measured at excitation frequency 0.5MHz for a population of identical polystyrene beads suspended in phosphate saline buffer.
**Figure 1b** Density plot of impedance magnitude versus impedance phase measured at excitation frequency 10 MHz for a population of identical polystyrene beads suspended in phosphate saline buffer.
**Figure 2a****.** Top view of microfluidic chip embodiment showing the alignment area and the detection area of the common microfluidic channel.
**Figure 2b****.** Detailed top view of a section of microfluidic chip showing the alignment area and the detection area of the common microfluidic channel.
**Figure 3a****.** Cross-section through the line A-A' of the microfluidic chip shown in Figure 2b.
**Figure 3b****.** Cross-section through the line B-B' of the microfluidic chip shown in Figure 2b.
**Figure 3c****.** Another embodiment of cross-section through the line A-A' of the microfluidic chip shown in Figure 2b.
**Figure 4a****.** Isometric view of the common microfluidic channel around the alignment area demonstrating effect of the field on the alignment of the particles.
**Figure 4b****.** Top view of the common microfluidic channel around the alignment area demonstrating effect of the field on the alignment of the particles.
**Figure 4c****.** Cross-section view of the common microfluidic channel around the alignment area demonstrating effect of the field on the alignment of the particles.
**Figure 5a** Top view of a segment of microfluidic chip comprising two guidance channels and one sample channel.
**Figure 5b** Cross-section through the line A-A' of the microfluidic chip shown in Figure 5a.
**Figure 5c** Cross-section through the line B-B' of the microfluidic chip shown in Figure 5a.
**Figure 5d** Cross-section through the line C-C' of the alignment area of the microfluidic chip shown in Figure 5a.
**Figure 6a** Top view of a segment of microfluidic chip comprising two alignment areas each equipped with two alignment electrodes.
**Figure 6b** Detailed top view of a segment of microfluidic chip comprising two alignment areas each equipped with two alignment electrodes.
**Figure 6c** Cross-section through the line A-A' of the first alignment area of the microfluidic chip shown in Figure 6b.
**Figure 6d** Cross-section through the line B-B' of the second alignment area of the microfluidic chip shown in Figure 6b.
**Figure 7a** Top view of a segment of microfluidic chip comprising a single alignment area equipped with two alignment electrodes of varying shapes and producing a field with varying direction along the common microfluidic channel.
**Figure 7b** Detailed top view of a segment of microfluidic chip shown in Figure 7 comprising a single alignment area equipped with two alignment electrodes of varying shapes and producing a field with varying direction along the common channel.
**Figures 7c****,****7d,7e** cross-section through the lines A-A', B-B', C-C' of the common channel shown in Figure 7b, all made perpendicular to the flow direction.
**Figure 8a** cross-section of the common channel with two alignment electrodes producing preferentially vertically alignment field and the sample fluid focused in the vicinity of the left wall of the channel.
**Figure 8b** cross-section of the common channel with two alignment electrodes producing preferentially horizontal alignment field at the sample fluid focused by the hydrodynamic focussing
**Figure 9a** cross-section of the common channel with two alignment electrodes producing preferentially vertically alignment field and the sample fluid focused at the vicinity of a left lower corner of the common channel.
**Figure 9b** cross-section of the common channel with two alignment electrodes producing preferentially horizontal alignment field and the sample fluid focused at the vicinity of a left lower corner of the common channel.
**Figure 10a, 10b****,** **10c****,** cross-sections of common channels with non-rectangular shape and various orientation of the alignment electrodes.
**Figure 11a****,** **11b** cross-sections of a common channels with the alignment electrodes separated from the interior of the channel by insulating layers and making electric contacts via contact windows formed on two opposite walls of the common channel.
**Figure 11c** cross-section of a common channel with the alignment electrodes separated from the interior of the channel by an insulating layer and making electric contacts via contact windows formed on one wall of the common channel.
**Figure 12** Cross-section of the common channel with the alignment electrodes electrically insulated from the interior of the common channel.
**Figure 13a****,** **13b** Cross-section of the common channel along the flow direction by a vertical z-y plane with another configuration of alignment electrodes

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

"Particulate" as applied to a particulate containing fluid means a solid body in the fluid or a semi-solid, i.e. a body with properties different to that of the fluid. Examples include particles of metals, oxides, nitrides, sulphides, polymer particles, particles of inorganic or organic materials, particles of gel, also composite particles, and mixed particles, nanoparticles, microparticles, particulate complexes, cells, clusters of cells, bacteria, fungi, virus, clusters of viruses, particles of proteins. Likewise, "particulate containing fluid" means a fluid containing particulates. Examples include cell containing fluids, such as sperm containing fluid.

"Analysis" means determining a qualitative or quantitative characteristic of the particulates in the fluid, for example determining whether the particulates are a homogenous population or a heterogenous population, determining the amount or concentration of particulates, or differentiating or sorting the particulates based on differences. Thus, the term broadly covers analysis of the particulates (i.e. cells) qualitatively or quantitatively, or differentiation or sorting of the particulates based on detected impedance response differences.

"Cells" means any type of cell, including mammalian cells such as sperm, white blood cells, red blood cells, bone marrow cells, immune cells, epithelial cells, nerve cells, pulmonary cells, vascular cells, hepatic cells, kidney cells, skin cells, stem cells, or bacterial and fungal cells and hybridomas, yeast cells, cancerous cells. Generally, the particulate containing fluid contains at least two different types of particulates, for example different cell types, sperm of different sex, sub-populations of the same cell types, the same cell type having different phenotypes, dead and living cells, diseased and non-diseased cells, immature and mature cells of the same kind. The apparatus and methods of the invention may be employed to analyse and/or differentiate and/or separate these different types or phenotype of particulates/cells.

"Different phenotypes" as applied to cells means different populations of cells (i.e. hepatic cells and vascular cells), different sub-populations of the same cell type (i.e. different types of cartilage cells), different phenotypes of the same cell type (i.e. cell expressing different markers, diseased and healthy cells, transgenic and wild-type cells, stem cells at different stages of differentiation).

"X and Y population" as applied to sperm cells means male sperm and female sperm cells.

"Focussed stream of particulate containing fluid" means a fluid containing particulates in the form of a focussed beam of particulates positioned within a guidance stream. In one embodiment the particulates in the focussed beam are focussed into a single cell stream arrangement. In one embodiment, in which the particulates have an anisotropic shape, particulates in the focussed beam are aligned in the same direction.

"Microfluidic chip" means a chip having at least one microfluidic channel having a cross-sectional area of less than 1 mm² and a length of at least 1 mm. In one embodiment, the microfluidic chip has at least one microfluidic channel having a cross-sectional area of less than 0.25 mm². In one embodiment, the microfluidic chip has at least one microfluidic channel having a cross-sectional area of less than 0.01 mm². In one embodiment, the microfluidic chip has at least one microfluidic channel having a cross-sectional area of less than 0.0025 mm². In one embodiment, the microfluidic chip has a plurality of microfluidic channels, for example at least 2, 3, 4, 5, 6, 7, 8, 9 or 10 microfluidic channels. In one embodiment, the microfluidic chip has at least one microfluidic channel having a length of at least 1.500 mm. In one embodiment, the microfluidic chip has at least one microfluidic channel has a length of at least 2 mm. In one embodiment, the microfluidic chip has a length of at least 3 mm. In one embodiment, the microfluidic chip comprises a plurality of layers, for example at least 2, 3, 4, 5, 6, 7, 8, 9 or 10 layers.

"AC impedance changes" should be understood to mean changes in impedance detected between the detection electrodes. The changes may include changes in amplitude, phase, or amplitude and phase of the signal.

"In electrical communication with the microfluidic channel" as applied to the electrodes means that the electrodes are in direct contact with the fluids analysed in the microfluidic channel.

"Detection zone" refers to a section of the microfluidic channel that comprises a sensor (i.e. detection electrodes) configured to detect a signal corresponding to a particulate passing the sensor (i.e. passing through the electrical field generated between the detection electrodes). The sensor is generally detection electrodes, although other sensors may be employed (i.e. optical sensors). When the sensor comprises detection electrodes, they typically comprise an energising electrode and a signal electrode operatively coupled to a detector (for example a lock-in amplified). The detection electrodes are preferably configured to detect particulates passing along the microfluidic channel by impedance spectroscopy as described above. The voltage V_{D} between the detection electrodes is generally less than the voltage between the alignment electrodes.

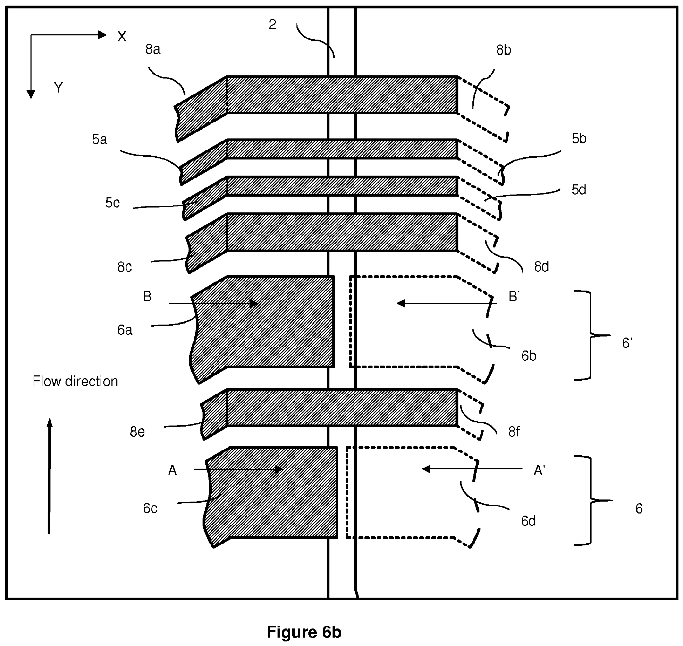

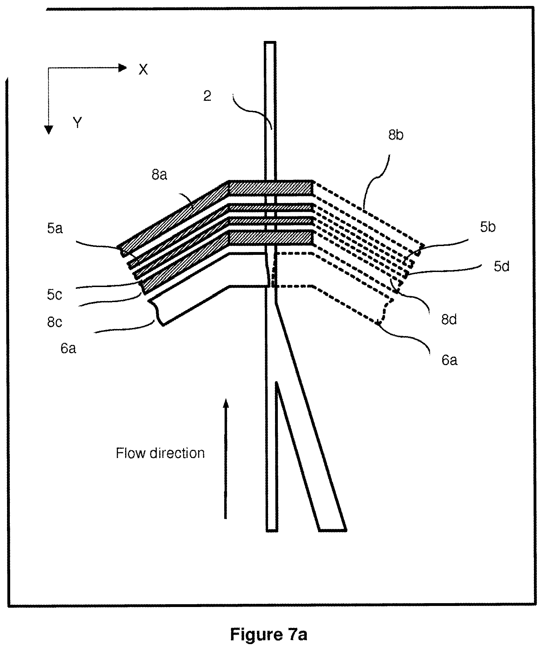

"Alignment zone" refers to a section of the microfluidic channel upstream of the detection zone that is configured to align anisotropic particulates in the microfluidic channel prior to the particulates reaching the detection zone. The alignment zone comprises alignment electrodes configured to generate an alignment electrical field capable of aligning the particulates into a common alignment. The voltage V_{A} between the alignment electrodes is generally greater than the voltage V_{D} between the detection electrodes. The alignment electrodes are preferably configured to generate an alignment electrical field that is non-uniform. The alignment electrodes are preferably configured to generate an alignment electrical field that is not aligned with the detection electric field, for example at an angle of 5-90 degrees to the detection electric field. The alignment electrodes are preferably configured to generate an alignment electrical field that is not aligned with the detection electric field, for example at an alignment angle of 5-90 degrees to the detection electric field. The alignment electric field may not be of the same AC frequency as the detection AC electric field. Alignment AC electric field may have a frequency such that complex dielectric permittivity of the particulates at that frequency is significantly different from the one of the fluid carrying these particles This may be done to increase an alignment torque and produce better alignment. In one embodiment, the alignment electrodes are configured to provide an alignment electrical field having an alignment angle that varies longitudinally along the microfluidic channel. In one embodiment, the alignment electrodes comprise a first (proximal) pair of alignment electrodes configured to generate a first alignment electrical field at a first alignment angle, and a second (distal) pair of alignment electrodes configured to generate a second alignment electrical field at a second alignment angle. This is illustrated in Figs 6b to 6d. Typically, the second alignment angle is greater that the first alignment angle. In another embodiment, the first alignment angle is greater that the second alignment angle. In another embodiment, the electrodes extend longitudinally along the alignment zone, and are configured so that the overlap between the electrode and the microfluidic channel varies longitudinally to provide an alignment electrical field having an alignment angle that varies longitudinally along the microfluidic channel. This is illustrated in Figs 7a to 7c. In one embodiment, the alignment electrodes do not comprise a signal detector. "Alignment electrical field" refers to an electrical field that is capable of aligning anisotropic particulates into a common alignment.

"Separation zone" is a part of the microfluidic chip, distal of the detection zone, where particulates in the fluid can be separated based on the AC impedance changes in the channel caused by the particulates and in accordance with the results of the characterization of the particulates in the detection zone. The separation zone generally includes a force generator operably connected to the electrode pair and configured to exert a force on the particulates in response to signals from the detection zone, to separate the one or more particulates from the stream of fluid. Examples of suitable force generators for use in cell sorting apparatus are well known in the art and described for example in [C.W. Shields IV, C.D. Reyes, G.P.Lopez, Microfluidic cell sorting: a review of the advances in the separation of cells from debulking to rare cell isolation, Lab. Chip 2015, 15(5) 1230-1240]. In one embodiment, the apparatus will typically include a processor operably connected to the at least one electrode pair and the force generator, and configured to actuate the force generator in response to a signal received from the electrode pair. The actuating signal may be pre-programmed into the processor, and may vary from cell type to cell type.

The term "anisotropic" refers to being not spherical in overall symmetry of particle's shape or its response to the stimulus used in the apparatus. In the simplest case, this refers to overall shape of the particle (cell). For example, if the particle is elongated, ellipsoidal, bar-shaped or disk-shaped, discoid, this is then described as anisotropic in contrast to a spherical shape particle that is being described as isotropic. However, the overall shape in its own right is insufficient to distinguish between anisotropic and isotropic particles (cells).

For example, if a conducting rod (segment of wire) is embedded into an insulating sphere, this forms an anisotropic particle even if the overall shape of the particle is spherical, i.e. isotropic. The reason is that such a particle has different response to the Radio Frequency (RF) electromagnetic field depending on whether it is directed with the length of the rod along the field or perpendicular to the field. The main response to the RF field will be in this case from the metallic rod, this response will be highly anisotropic, the insulating spherical envelope will have little effect on the situation. The same applies to optical response: it will be different depending on the direction of the light incidence and the polarization with respect to the long axis of the rod, again the effect of the isotropic dielectric envelope on the optical response will not alter anisotropic response from the conducting rod. The same applies to the cells. The main contribution to RF signal response from a cell may not come from the exterior periphery of the cell but from its interior features. This depends on the structure of the cell and the RF frequency.

When referring to laminar flow regime, we shall imply the flow conditions that fall under the Stokes regime (∼1<Re<∼1000). Re is the Reynolds number defined as Re=ρUH/µ, where ρ, U and µ, are the fluid density, the average velocity and dynamic viscosity respectively and H is the characteristic channel dimension. In some cases the effect of particle focusing may still be achieved when Reynolds number is below 1 and therefore the invention is not restricted to the situation of ∼1<Re<∼1000. Generally the range of Re values at which the focusing is achieved, also depends on the difference between the density of the liquid and the density of the particles. The greater is the difference, e.g. the heavier are the particles compared to the liquid, the greater is the effective gravity force (difference between the gravity force and the buoyance force) pulling the particles down from the locations defined by the hydrodynamic forces. Therefore, the greater is the difference between the densities, the greater should be the force bringing the particles towards hydrodynamically favoured positions to achieve effective focusing of the particle's trajectories.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

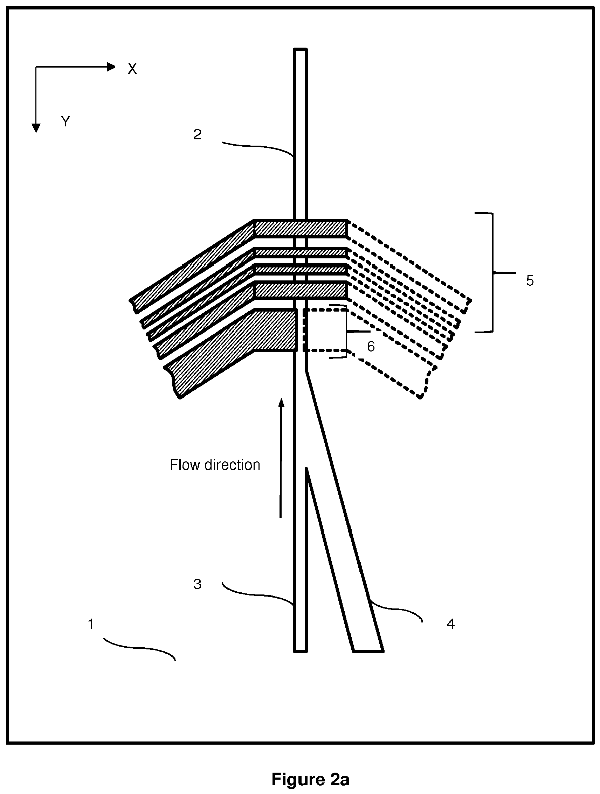

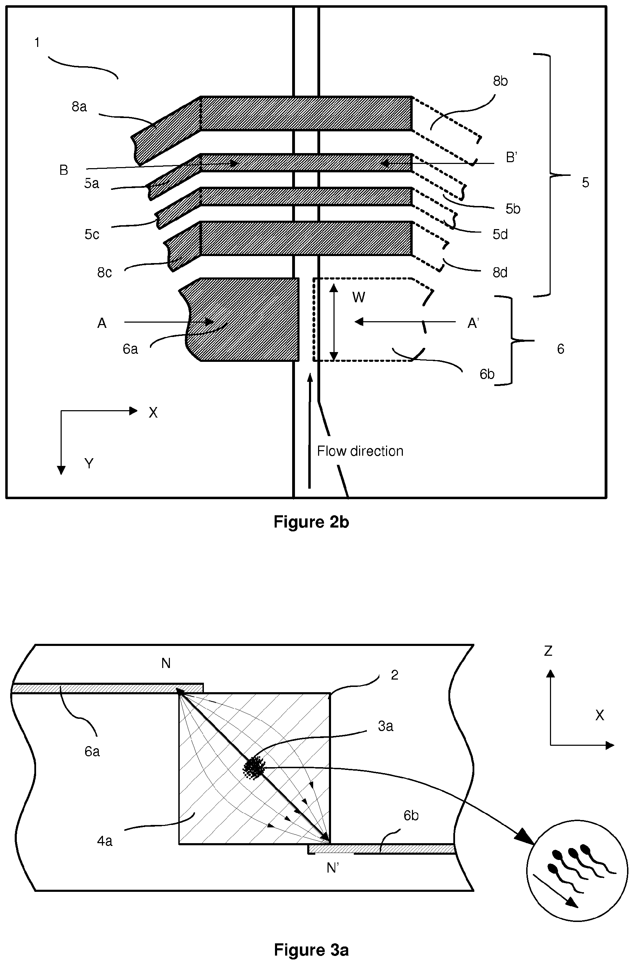

Figure 2a describes embodiment of a section of microfluidic chip 1 comprising the common microfluidic channel 2, sample fluid channel 3 and guidance fluid channel 4 having the detection area 5 and the alignment area 6. There can be other elements of the microfluidic chip such as separation area where the cell/particles are separated according to their type and the area. There are different configurations where a number of channels containing sample fluid and the guidance (sheath) fluid merge into the single common channel. Figure 2a is only one of the embodiments of such merging but other possible embodiments are shown in other figures of this document. In this embodiment shown in Figure 2b, the common microfluidic channel 2 has a rectangular cross-section with the dimensions in the following range: width 20 to 3000 micrometres, more preferably 20 to 500 micrometres and the height in the range of 10 to 3000 micrometres, more preferably 20 to 500 micrometres. The cross-section of the common microfluidic channel 2 does not need to be rectangular, and channels with other cross-sections can also be deployed. The examples of such channels will be given below. The alignment area 6 is equipped with the alignment electrodes 6a,6b. The detection area 5 is equipped with the detection electrodes 5a,5b,5c,5d. The detection electrodes are not labelled in Fig. 2a but these are sketched in the same way as in Fig. 2b. The alignment electrodes 6a, 6b are connected to the voltage and/or current generator/generators. The electrodes are connected to different potentials (voltage points) of the generator so that a potential (voltage) difference is created between the alignment electrodes. The detection electrodes 5a,5b,5c,5d are connected to the detection circuit. In this circuit some detection electrodes for example 5a and 5c are connected to AC voltage or current generators and others 5b and 5d are connected to the AC voltage or current detectors. In the preferred embodiment the voltage or current detectors are phase sensitive detectors also known as lock-in amplifiers commonly used in AC impedance measurements. These are not described here for shortness and known for skilled in the impedance spectroscopy art. We shall only stress that some of the detection electrodes are connected to excitation AC voltage or current sources even though we attribute them to the subset of detection electrodes and other detection electrodes, are connected to AC voltage or current detectors tuned to the frequency or frequencies of these detection electrodes. The walls of the microfluidic chip could be made of plastic, glass or other materials, e.g. silicon. The alignment and detection electrodes are made of metal, these could be e.g. gold, copper, silver electrodes of these could also be made out of other metals that have special bio-compatibility properties and enhanced resistance to corrosion such as palladium, platinum, tantalum, titanium, etc. The thickness of the electrodes may be in the range 0.1-10 micrometers. The electrodes could also be composed of several layers, for example a sub-layer of metal A serving the purpose of enhanced adhesion to the walls of the channel followed by a layer B deposited on the surface of the layer A. These details are well appreciated by those skilled in the art of making film electrodes for electronic and electrical applications. The alignment and the detection electrodes preferably are open to the interior of the common channel 2 to make electric contact with the fluid inside the channel. One could also construct embodiments where the electrodes make electric contact with the common channel only along a section of the area of the electrode being in mechanical contact with the wall of the channel while keeping the rest of the electrode electrically insulated from the common channel. These embodiments will be described below.
Figure 2b shows details of a top view of the common microfluidic channel 2 with the flow direction being parallel to the plane of the drawing. The flow direction is indicated on the drawing, and it is such that the alignment area 6 precedes the detection area 5 when moving along the flow direction in the common microfluidic channel 2. The chip could also be equipped by the separation area that is located downstream from the detection area 5. The separation area is not shown in Figure 2a or Figure 2b. Figure 2b also shows two pairs of electrodes connected to a fixed (e.g. ground) potential. These electrodes are positioned at the two opposite surfaces of the channel and marked by the numerals 8a,8b,8c and 8d. These electrodes are optional and they serve to reduce the noise and the voltage induced in the detection electrodes from various other electrodes of the microfluidic chip and various other sources of noise. We will refer to these electrodes as shielding or grounding electrodes. The flow of fluids in the channels is sustained by a pump or a pressure source. This could be e.g. one or several UniGo pressure driven pumps from Cellix Limited, Dublin, Ireland. The pump/pumps are not shown in figures of this document for shortness and those skilled in the art of microfluidics are familiar with this aspect.

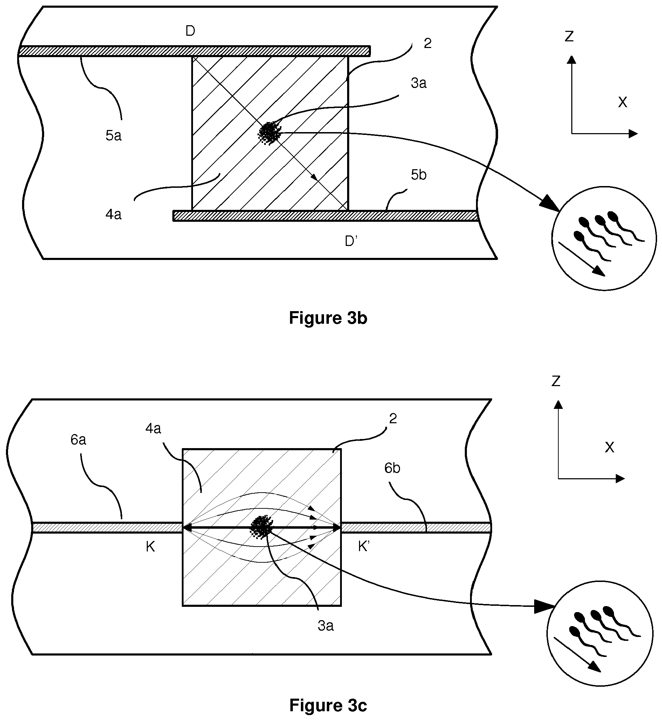

The cross-section of the common microfluidic channel along the lines A-A' and B-B' of Figure 2b, is shown in Figures 3a and 3b respectively. There is a flow of the particles containing fluid 3a enveloped in the flow of the guidance fluid 4a. For shortness, we may call the particles-containing fluid also the sample fluid. These two terms may be used interchangeably. We shall also refer to the liquid carrying particles/cells. Therefore it is understood that the liquid carrying particles/cells and the particles/cells themselves form sample fluid. For example, this could be TRISA based buffer which is commonly used for holding sperm cells which might contain concentration of sperm cell from 0.1-100 million sperm cells per millilitre. To obtain particles containing fluid 3a within the flow of guidance fluid 4a, one can employ hydrodynamic focusing extensively described in the state-of-the-art The particle containing fluid 3a is the fluid that contains the particles of interest. These could be organic or inorganics particles. These could also be alive or dead cells including mammalian cells, sperm cells, yeast cells, particles of biological and non-biological origin etc. In this embodiment the particles-containing fluid stream is located at the centre of the guidance fluid 4a flow, i.e. at the centre of the cross-section of the common microfluidic channel 2. Therefore, the guidance fluid 4a performs the function of the sheath fluid focussing the flow of the particle containing fluid into a tighter flow of reduced cross-section. It will be appreciated by those familiar with hydrodynamic focussing that there is no sharp physical boundary between the sample fluid flow and the guidance fluid in the common channel. These two liquids gradually intermix by diffusion and under influence of other forces, along the common channel as we move from the point of their mergence downstream. However, over the distance of 0.1-10 millimetres one could readily consider the flow as the flow of two fluids: the sample fluid enveloped by a guidance fluid. The guidance fluid 4a does not need to envelope the sample fluid all around. One could construct hydrodynamic focussing where the sample fluid is focussed into a corner of the common channel cross-section or close to one of its walls. In these embodiments the guidance fluid envelopes the sample fluid at some sides of the sample fluid flow. Some of these embodiments will be sketched and described below. The guidance fluid 4a may be interchangeably called the sheath fluid in this document. To obtain the focussing of the particles-containing fluid 3a by means of the guidance fluid, the microfluidic chip 1 comprises sample microfluidic channel 3 sustaining a flow of particles containing fluid and a guidance microfluidic channel or channels 4 sustaining a flow of guidance (sheath) fluid. The two channels merge forming the common microfluidic channel 2 so that the sample microfluidic channel 3 is coupled to the centre of the guidance microfluidic channel 4.

Typically the flow rate of the guidance fluid is substantially greater than the flow rate of the sample fluid, i.e. greater by a factor of 2 to 100. The linear velocity of the liquid in the sample microfluidic channel and the guidance channel is in the range 0.01 meters/sec to 10 meters/sec. Those skilled in the art appreciate that the linear velocity varies strongly across the channel and normally is greatest at the centre of the channel at least for pressure driven flows. The cross-section dimensions of the guidance channels and the sample microfluidic channel are in the same range as those of the common microfluidic channel described in relation to Figure 2a. The flow rates in the channels can be readily calculated by those skilled in the art given the linear velocities of the flows and the area of the channels cross-section.

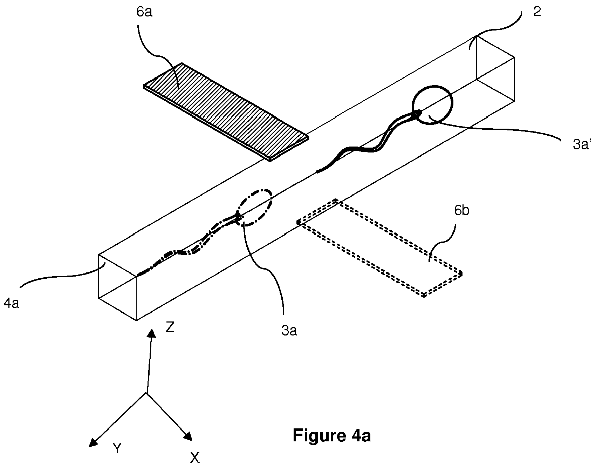

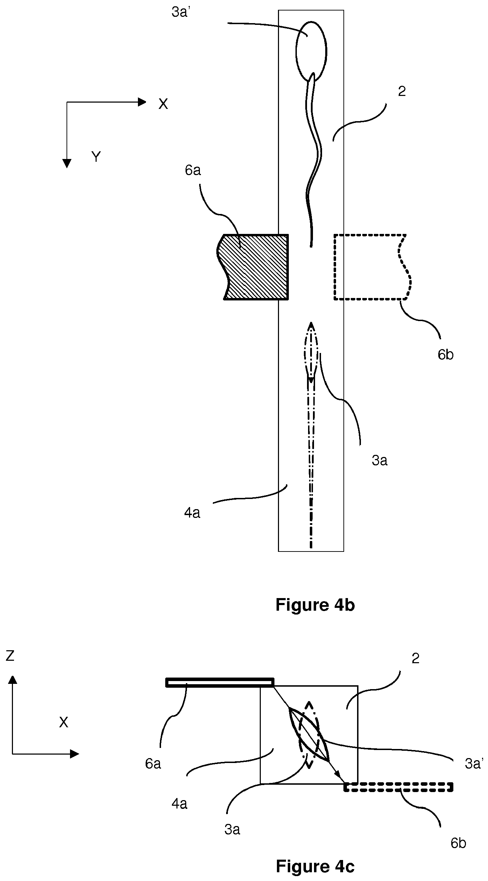

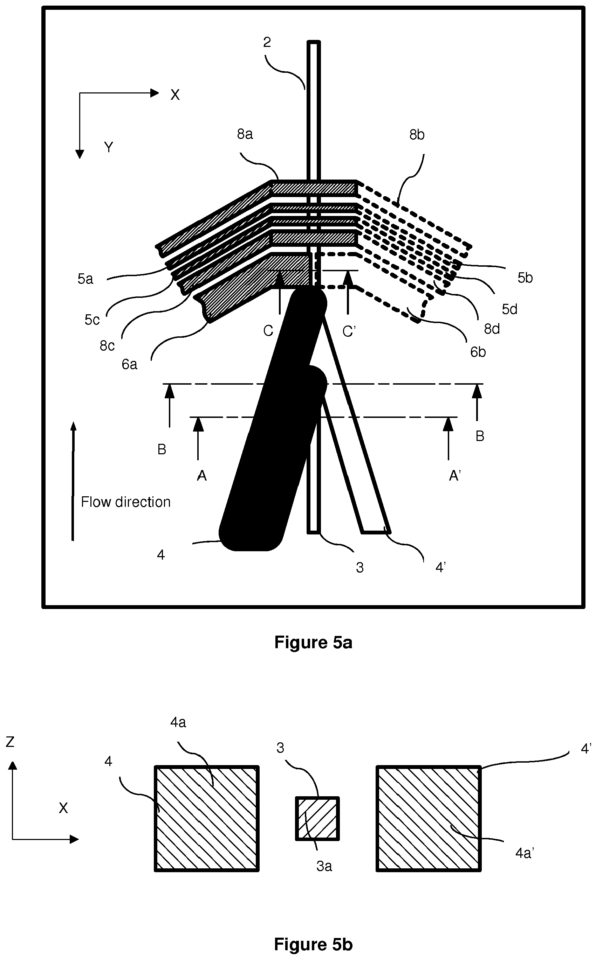

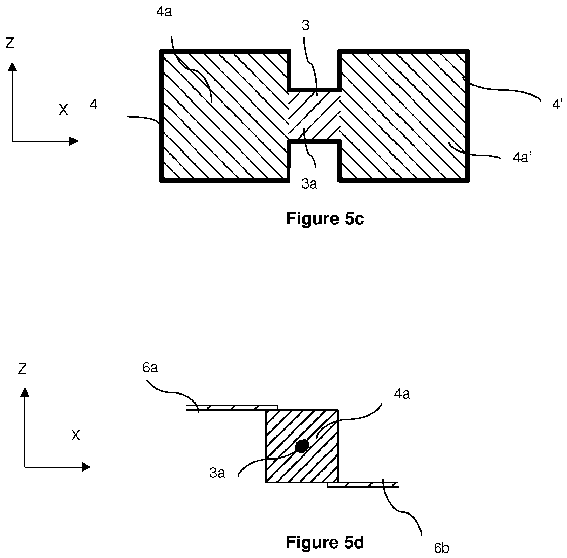

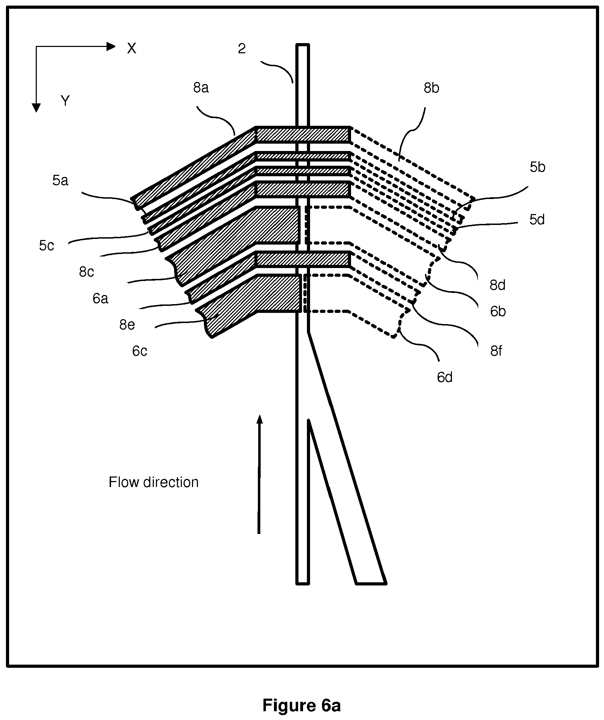

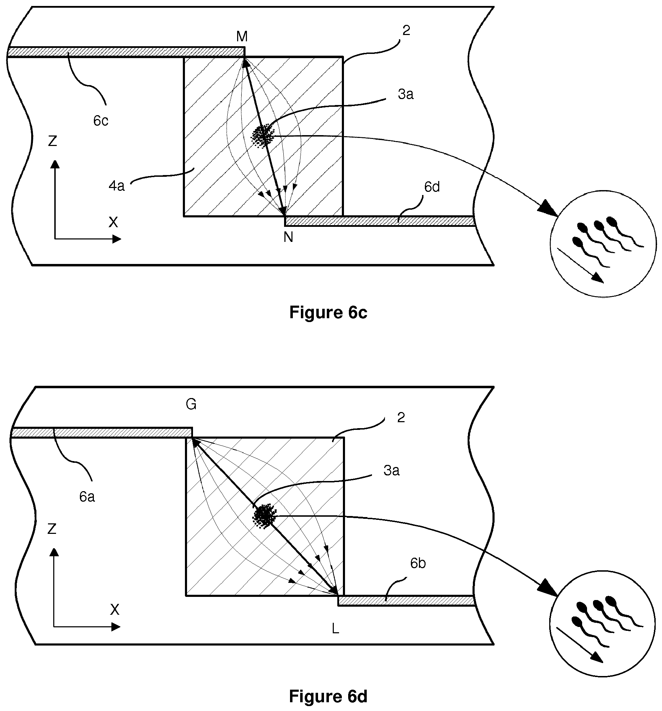

Another configuration achieving such merging of the sample microfluidic channel and the guidance channel, is shown in Figure 5a. In this configuration there are two guidance channels, 4 and 4', on each side of the sample microfluidic channel. Figure 5a shows the top view of the channels. Figures 5b, 5c and 5d show the cross-sections through the lines A-A', B-B' and C-C' respectively, all perpendicular to the direction of the flow. This configuration allows maintaining the particles-containing fluid mostly at the centre of the common microfluidic channel in the detection area of the microfluidic chip. Furthermore, with the correct ratio of the flows in the guidance channels 4 and 4' and the sample microfluidic channel 3, and the correct ratio of the cross-sections of the guidance channels 4 and 4'and the common microfluidic channel 2, the flow of the particles-containing fluid 3a gets compressed by the sheath (guidance) fluid 4a and 4a'. This is known as hydrodynamic focussing. The focussed flow of the particles containing fluid gradually becomes defocussed due to the diffusive movement of the particles perpendicular to the flow direction. The detection area described in Figure 5a is located at short enough distance away from the point where the sample microfluidic channel merge with the guidance channels, at such a distance where the particles containing fluid still remains focussed at the centre of the common microfluidic channel 2. This distance could be in the range 20 to 20000 micrometers. The configuration in the Figure 5a has alignment area equipped with alignment electrodes 6a and 6b and detection area equipped with detection electrodes 5a, 5b, 5c and 5d and optional grounding electrodes 8a, 8b, 8c and 8d.
To explain the operation principle, we will refer back to the configuration shown in Figures 2a and 2b. Figure 3a shows the cross-section perpendicular to the direction of the flow across the line A-A' indicated in Figure 2b. The two alignment electrodes 6a and 6b are terminated such that the edges of the electrodes marked by the letters N and N', are positioned at the proximity of two opposite corners of the common microfluidic channel 2. The width of the electrodes along the length of microfluidic channel marked by the letter W in Figure 2b, is in the range of 0.1 to 30 mm. The alignment electrodes 6a and 6b are connected to a voltage source that is not shown in Figure 2b for brevity. The voltage source can generate either a DC voltage or an AC voltage with the frequency F1 up to 100 MHz between the alignment electrodes 6a and 6b. Preferably, if alignment electrodes 6a and 6b are energised by AC voltage, its frequency is chosen in the range of 50 KHz to 10 MHz. The lower value of the AC frequency is selected such that the time required for the particles/cells to travel across the alignment area is substantially greater than the period of the AC voltage F1 that is calculated as 1/F1. The upper value of the frequency F1 is selected such that the voltage can be coupled to the alignment electrodes 6a and 6b without major losses. Generally, this voltage can be readily coupled at frequency up to 100-200 MHz. The coupling of voltage can also be achieved for AC voltage at even higher frequency but this can be associated with further issues of AC electronic engineering such as impedance matching, standing waves, etc. The voltage applied between the alignment electrodes 6a and 6b is in the range of 0.1-100 V DC or AC and more preferably in the range of 0.5-20 Volt although the values outside this range are also possible. The voltage of this value is applied between the two alignment electrodes. The AC voltage applied to the alignment electrodes does not need to be sinusoidal. One could also consider other time dependencies of the voltage, e.g. a meander type dependency changing rapidly between two values of voltage, or other functions of voltage against time. The voltage applied between the two alignment electrodes generates DC or AC electric field, and the lines of the electric field pass through the centre of the common microfluidic channel as shown in Figure 3a, and therefore they also pass through the flow of the particles-containing fluid that is flowing through the centre of the common microfluidic channel.
The direction of the field passing through the particles containing fluid is along the line NN'. If the particles contain no net charge, or very small net change, the electrophoretic force acting on them is negligibly small. This shall be the case for many fluids containing suspensions of cells. The electrophoretic force could be significant in other cases, e.g. for the case of ions in liquid/electrolyte. However, this will not be a dominant force in the case of much larger particles or cells. Such larger particles or cells may contain surface charges distributed along the cell but not a significant net charge relative to the volume of the particle or cell. The electric field this could also create dielectrophoretic force acting on the particles even if it has not net charge. The dielectrophoretic force results from the distribution of the surface charges on the particle (cell). However, the dielectrophoretic force shall be rather small as it scales up with the gradient of the electric field. As the gradient of the field at the centre of the common microfluidic channel as shown in Figure 3a is virtually zero, the net dielectrophoretic force acting on uncharged particles and cells will also be virtually zero. This is not the case in relation to the torque. If the particles are spherically symmetric, there will be no net torque resulting from this electric field. However, if the particles are not spherically symmetric, there will be a torque acting on these. For example, in the case of cells that are discoid in shape, the long axis of the cell will align along the field direction. For a cell/particle that is elongated in one direction, such as e.g. a sperm cell, the torque will act to align the elongated direction along the direction of the field.
Zoom-in into the flow of the particles containing fluid is shown in Figures 4a, 4b,4c. Figure 4a displays isometric view of the common channel 2 and schematic of alignment of sperm cell 3a (dashed line) after passing alignment electrodes 6a and 6b which is indicated by rotated sperm cell 3a' (solid line). Alignment of sperm cells 3a' is observed along the direction of the field between alignment electrodes 6a and 6b. Such uniform alignment of the cells reduces the spread of the signal from the individual cells in the detection electrodes and this increases the ability of separation of cells in different subsets, e.g. separation into X and Y- sperm cells. This alignment does not necessarily require that the particles/cells have anisotropic shape. They could be for example spherically symmetric in shape but yet have anisotropic dielectric permittivity due to the internal structure of the particle/cell. In this case there will be a torque acting on the particle/cell due to the anisotropy of the dielectric permittivity. It should be appreciated that the dielectric permittivity ε(f) of a typical cell is frequency f dependent. It may also depend on other external factors such as pH liquid or temperature. It is preferable to choose the AC frequency f of the voltage energising the alignment electrodes 6a and 6b such that the dielectric permittivity ε at this frequency is significantly different from that of the liquid where the cells are placed. For example, if the frequency is such that the dielectric permittivity of the liquid carrying the particles/cells is the same as that of the particles themselves, then there will be no net torque acting on the particles. The dielectric permittivity is a complex function that has two components: the real Re ε(f) and imaginary Im ε(f) ones, both of these being functions of frequency. It is preferable to select the frequency such that the real part of the dielectric permittivity Re ε(f) of the particle (cell, sperm cell) is as different from the same component of the liquid carrying such particles, as possible. The dielectric permittivity of the sheath fluid may differ from that of the particle containing fluid. In this case the primary concern is to ensure that there is difference between the dielectric permittivity's of the particle/cells and that of particle carrying fluid.
The effect of the torque is to force the cells to form a more uniform alignment with respect to the detection electrodes. This is schematically shown in Figure 3b that shows the section along the line B-B' of Figure 2b. The detection electrodes 5a, 5b, 5c and 5d detect the change in impedance between them resulting from the cells passing through the detection area in between the detection electrodes. The signal detected by the detection electrode depends on the position of the cells with respect to the electrodes and also in the case of anisotropic cells, it depends on the cell orientation with respect to the electrodes. Generally, in the case of the discoid cells, the signal on the detection electrodes, i.e. the complex impedance change due to the passing cell, is greater if the cell has the plane of the greater cross-section aligned along the detection electrodes 5a and 5b. In the configuration of the Figure 3b, the signal on the detection electrodes is greater if the discoid cell has short axis aligned along the z-direction. The cells shown schematically in Fig. 3b are sperm cells. In the contest of the above discussion it is clear that the in the case of sperm cells thy need to be aligned with long axis long the X axis to maximise the signal. However, it is also clear in the contest of the previous discussion that the prime goal is not maximising the signal but achieving identical signal from identical cells so that they could be correctly identified and this means aligning the cells as much as possible along a single direction, regardless what that direction is. As the value of the signal detected by the detection electrodes is not only dependent on the type of the cell but also on its position and the alignment, it is crucial to achieve as uniform as possible a positioning and alignment of the cells (particles) with respect to the detection electrodes. This is necessary for the differentiation between different types of cells (particles) that is important for identification of different cell populations and also for the separation of different sub-populations of cells (particles). In the case of elongated cells such as e.g. sperm cells, the torque acting on the cells from the alignment electrodes aligns cells along the line D-D', that is approximately the line of the electric field in Figure 3a. The more homogeneous alignment of all the cells with respect to the detection electrodes reduces the spread of the values of the signals detected by the detection electrodes resulting from the single cells passing in between the detection electrodes.
The alignment of the cells could be achieved with various other configurations of alignment electrodes. One alternative alignment is shown in Figure 3c. In this case the alignment filed is horizontal, i.e. along the line K-K' and the discoid cells will turn to have their planes along the line K-K'. If the cells (particles) are elongated along one direction such as e.g. sperm cells, they will align their long axis along the x-axis. The direction of the alignment of the cells/particles is different in the embodiment of the alignment electrodes presented in Figure 3c and Figure 3b. However, it is important that in both of these cases, we achieve more uniform alignment of anisotropic cells/particles with respect to the detection electrodes and this reduces the spread in the electric signals produced by the particles making identification of the cells/particles more accurate.
It is desirable in some circumstances to have the frequency of the AC voltage applied to the alignment electrodes different from the frequency or frequencies at which the detection of the signal at the detection electrodes, is taking place. This can reduce the parasitic signal coupling from the alignment electrodes to the detection electrodes. To reduce the parasitic signal, other means could be also deployed such as a set of additional electrodes connected to a ground potential or another fixed potential positioned downstream from the alignment electrodes and before the detection electrodes. This is similar to the electrodes 8a, 8b, 8c and 8d connected to the ground potential shown in Figure 2b positioned in between the alignment area and the detection area.
Furthermore, the detection area could be comprised of a number of detection electrodes. For example, this could be two or more electrodes energised at one or multiple frequencies and two or more electrodes detecting these signals. Likewise, the alignment area could comprise more than two alignment electrodes. Each alignment electrode could be comprised e.g. of two or more conducting areas and although technically these are different electrodes, we shall treat this as one electrode and not discuss these trivial variations in greater detail. Instead, we shall consider more complex situations.
The embodiment comprising two pairs of alignment electrodes is shown in Figures 6a, 6b, 6c and 6d. Figure 6a shows the top view of the sample channel with the alignment area comprising two sets of alignment electrodes composed of four electrodes 6a, 6b, 6c and 6d , a set of shielding electrodes 8a, 8b, 8c, 8d, 8f, and 8e, one on each, the upper and the lower surface of the channel and a set of detection electrodes 5a, 5b, 5c and 5d . Figure 6b shows detailed view of alignment and detection areas of the microfluidic chip. Figure 6c shows cross-section A-A' perpendicular to the direction of the flow with the alignment electrodes 6c and 6d. These electrodes protrude along the upper and lower surface of the sample channel to a greater extent that the alignment electrodes 6a and 6c shown in Figure 6d, i.e. the line MN of Figure 6c forms lesser angle with the vertical line than the line GL of Figure 6d. In this embodiment, both lines, MN and GL, pass through the centre of the common channel 2 and yet they are not parallel to each other. Let us consider the effect of these two sets of alignment electrodes on elongated cells/particles. The effect of the first set of alignment electrodes 6c and 6d shown in Figure 6c, on the anisotropic cells/particles passing through the centre of the common channel 2 is rotate them so that the longer axes of the cells/particles align along the axis MN. However, this will not rotate those cells/particles that entered the area between the alignment electrodes 6c and 6d with their long axes being perpendicular to the axis MN and these will remain unaffected by the field created by the alignment electrodes 6c and 6d. Therefore, on the exit from the alignment area 6 comprised of alignment electrodes 6c and 6d, most cells/particles will have their long axis aligned along the line MN with the exception of a group of cells/particles that entered into the alignment area 6 with their long axes perpendicular- or nearly perpendicular to the line MN.
The embodiment shown in Figure 6b also has an electrodes 8f and 8e separating the alignment electrodes 6c and 6d from the alignment electrodes 6a and 6b that is connected to the ground potential. This is optional feature to reduce the current flow between the two sets of the alignment electrodes. The alignment electrodes 6c and 6d could be excited at a different frequency from the alignment electrodes 6a and 6b. However, they could also be excited at the same frequency. If the two sets of alignment electrodes are excited at the same frequency and at the same phase, e.g. by being excited from the same AC voltage generator, this could also reduce the current flow between the alignment electrodes 6c, 6d and alignment electrodes 6a, 6b. In the embodiment shown in Figure 6b the part of the common channel 2 proximal alignment electrodes 6c and 6d therefore forms the alignment area 6 and the part of the common channel 2 proximal alignment electrodes 6a and 6b forms the alignment area 6'. As will be appreciated by those familiar with the concept of electric field, the direction of the electric filed line shown in Figures 6c and 6d with arrows, is notional. With an AC field it will vary with the same frequency as the voltage applied to the alignment electrodes. The direction is merely indicated to distinguish field lines from other lines in the drawing. The same comments apply to several other figures in this document where the field lines are marked by arrows.

Once these cells/particles exit the alignment area 6 they enter into the alignment area 6', where they will be affected by the electric field directed along the line GL that is not parallel and not perpendicular to the line MN. In the embodiment shown in Figure 6d, there is an angle of some 20-45 degrees between these two lines. Embodiments with other values of the angle could also be used. This means that the electric field in the alignment area 6' will rotate both groups of cells: the ones that have their long axis nearly parallel to the line MN and the ones with their long axis perpendicular to MN, the latter is the group that was not affected by alignment filed of the alignment electrodes 6c and 6d. The same consideration applies to discoid cells: the presence of two alignment fields ensures that all the cells/particles are rotated under the influence of the two torques acting on the cells/particles in succession, and none of the anisotropic cells/particles remain unaffected by the alignment field regardless of their initial alignment at the point of entry into the first alignment area.

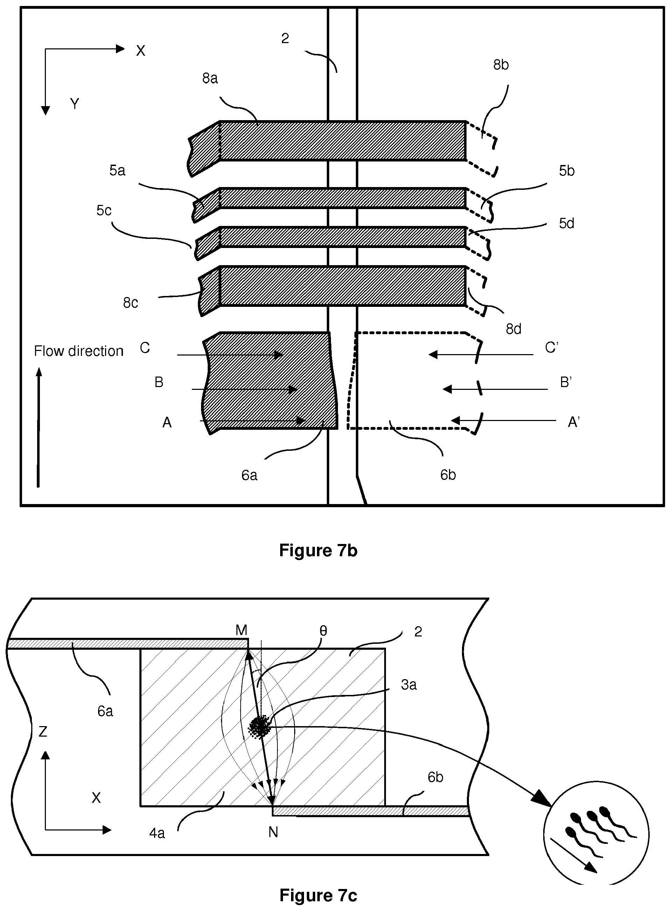

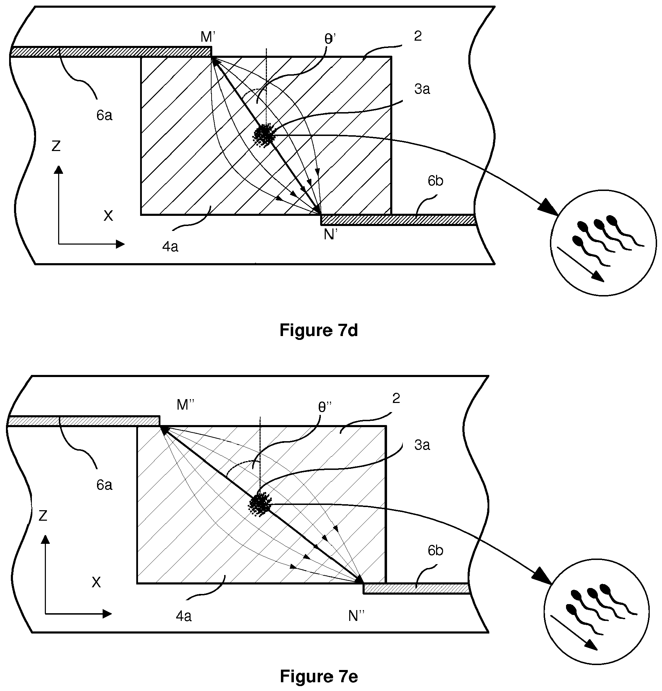

One could also construct an embodiment with one set of alignment electrodes with a continuously changing direction of the field. This is shown in Figures 7a, 7b, 7c, 7d and 7e. Figure 7b shows the top view of the segment of the common channel along the alignment area. The alignment electrodes marked as 6a and 6b have their right and left edges respectively curved as shown in Fig 7b. These are the edges that protrude over the common channel 2. This is shown by the cross-sections in Figures 7c, 7d, 7e corresponding to the lines A-A', B-B' and C-C' respectively. The edges of the alignment electrodes 6a and 6b are marked by the points M, M', M" and N, N', N" in Figures 7c, 7d, 7e respectively. The angle between the line MN and the vertical line in Figures 7c, 7d, 7e gradually changes along the length of the common microfluidic channel. This angle is marked as θ, θ', θ" in Figures 7c, 7d, 7e respectively. These two alignment electrodes of varying shape produce the result on the anisotropic particles/cells broadly similar to the one with two sets of alignment electrodes shown in Figures 6: the configuration with varied direction of the field along the length of the common channel ensures that the cells become aligned by the field regardless of the initial direction of the cell alignment at the point of entry into the alignment area.

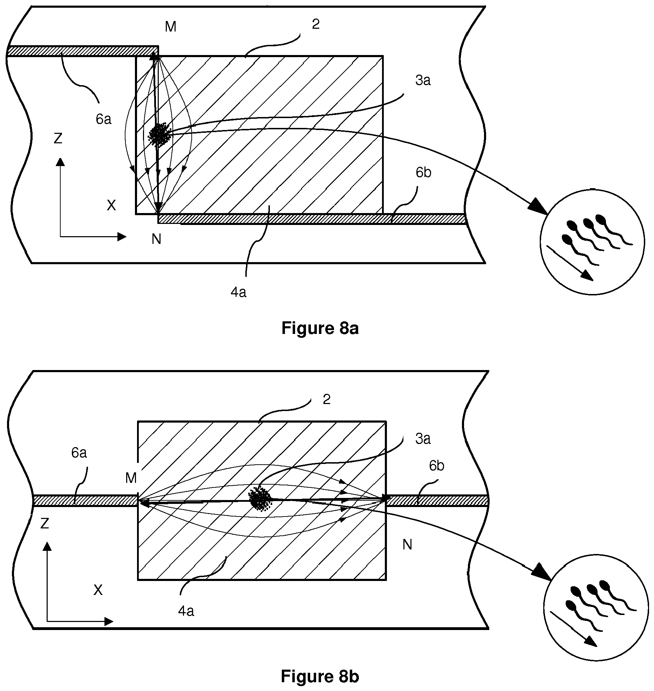

The sample fluid 3a does not need to be focused at the centre of the common channel 2. Figures 8a and 8b shows embodiment with the common channel 2 of rectangular cross-section with the sample fluid 3a located close to the vertical wall of the common channel 2. Figure 8a shows embodiment with two alignment electrodes 6a and 6b producing vertical or nearly vertical alignment electric field and Figure 8b shows embodiment with two alignment electrodes 6a and 6b producing horizontal or nearly horizontal alignment electric field. Both, Figures 8a and 8b, show cross-sections through the common channel 2 passing through the alignment area 6 of the common channel 2. We should point that Figure 8a shows the embodiment where the sample fluid 3a is not surrounded by the guidance fluid 4a at all the sides. In this the sample fluid 3a is making contact with the left wall of the channel 2 and the other sides of the sample fluid 3a are surrounded by the guidance fluid 4a. Most other embodiments shown in this document show the examples of the common channels 2 having the sample fluid 3a being enveloped on all the sides by the guidance fluid 4a. This is not a necessary requirement.

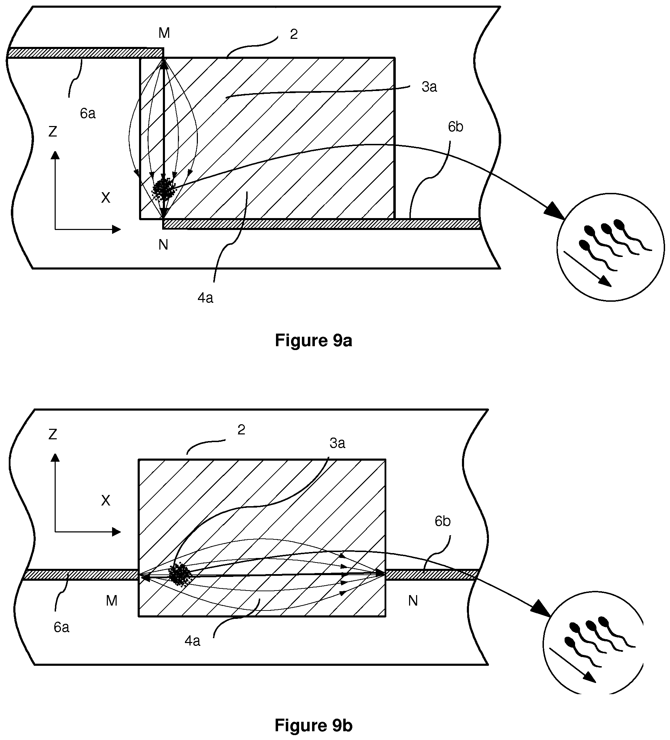

Figures 9a and 9b shows embodiment with a channel of a rectangular cross-section with the sample fluid positioned close to one of the corners of the rectangle. Figures 9a and 9b show the embodiment with the vertical (near vertical) and horizontal (near horizontal) alignment field directions respectively. The coupling of the guidance channel 4 and the sample microfluidic channel 3 as required to achieve positioning of the sample fluid 3a outlined in Figures 8a,b and Figures 9a,b, is not shown here. The embodiments with more than one set of alignment electrodes described with reference to Figure 5 and the embodiment with electrodes of a shape varying across the channel producing electric field of varying direction across the channel described with reference to Figure 6, can readily be constructed and are not described here for shortness.

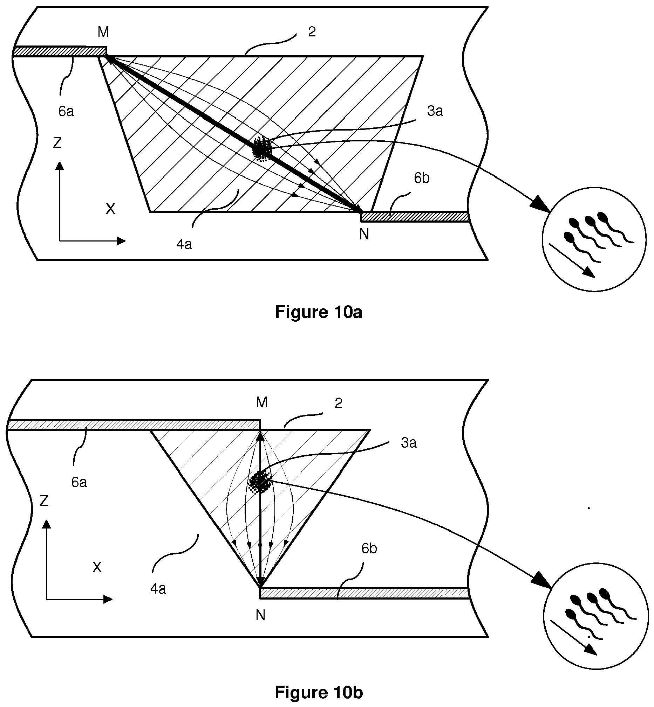

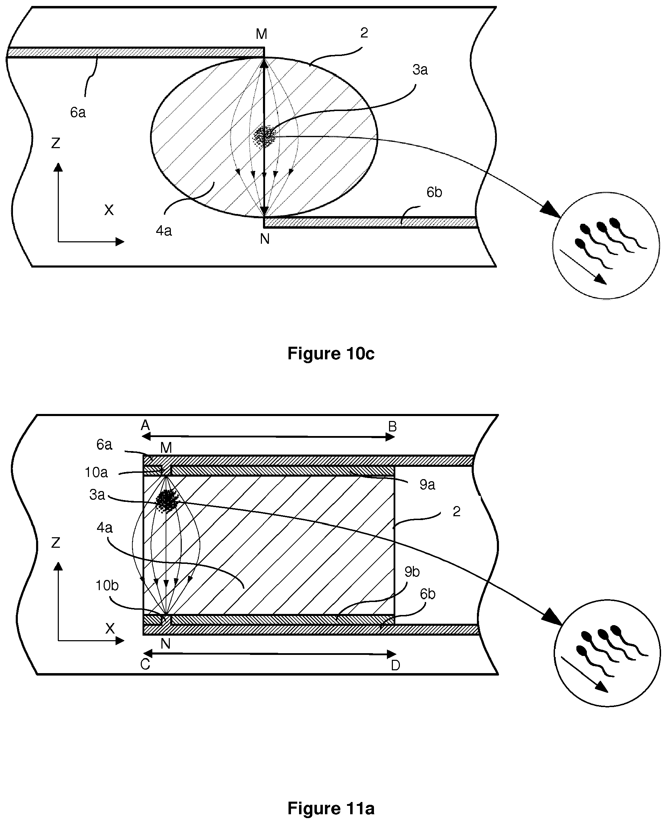

The common channel does not need to be of rectangular cross-section. Figures 10a,b,c, show the embodiments with non-rectangular cross-sections. Each of these can be equipped with horizontal (near horizontal), vertical (near vertical) alignment electric field. The embodiments could be equipped with tilted alignment electric field making any desired angle with the vertical axis. They could also be equipped with more than one set of alignment electrodes such as described with reference to Figure 5 and they could also be equipped with the electrodes of varying shape providing electric field of varying direction along the common channel such as the one described with reference to Figure 6.

The optimal direction of the alignment field is usually not only determined by the position of focused sample fluid within the common channel and the positions of the detections electrodes. This may also depend of the preferential orientation of the cells at the point of entry into the alignment area of the common channel. The matter is such that cells/particles having anisotropic shape may obtain preferential orientation due to hydrodynamic focussing. The anisotropic alignment of the cells/particles depends of the configuration of the channels: the sample microfluidic channel and the guidance channel, the cross-sections of these, the configuration of the merging of these channels into the common channel, the flow rates in the microfluidic channel and the guidance channel. It may be preferable to apply the alignment field in such a direction that it aligns cells along their preferential direction that cells have at the point of entry into the alignment area. This will reduce the angular spread in particles alignment and therefore reduce the spread in values obtained in the detection area from a single group of cells/particles.

Figures 10a, 10b and 10c describe common channels of various cross-sections. Only some examples of more simple channels are shown. Other configurations may include many other 2D shapes. In the same way, the sample channel and the guidance channel shown in Figure 4b could also have various other shapes. The need for channels of these non-rectangular shapes may arise in order to achieve the non-anisotropic alignment of the cells resulting from the hydrodynamic focussing in such channels.

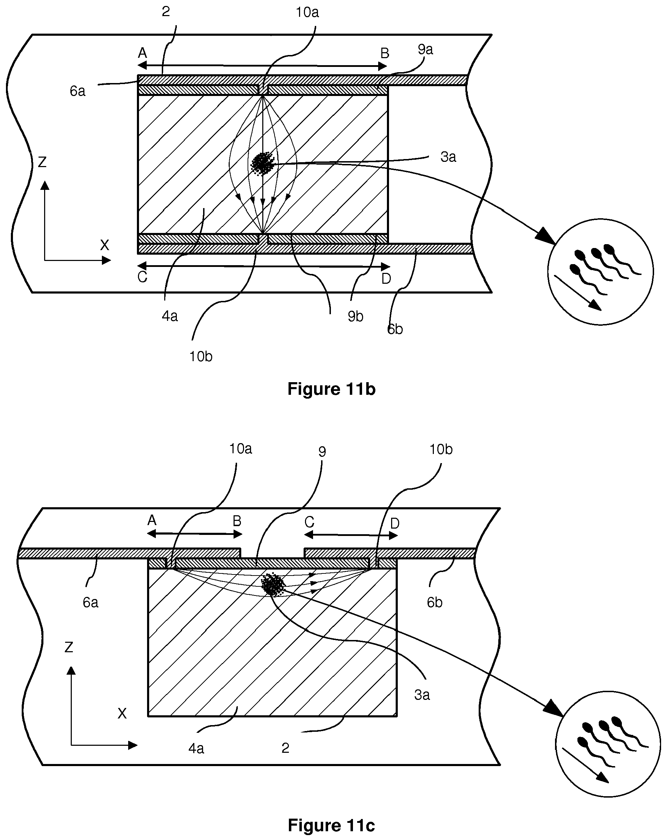

Figures 11a, 11b and 11c show cross-sections of common channels made across the alignment area and the alignment electrodes, like many other figures in this document. Figures 11a, 11b and 11c show embodiments where the alignment electrodes are positioned on the walls of the common channel. The difference between these embodiments and other ones described earlier is that the alignment electrodes are not directly open to the interior of the common channel but rather are positioned over an insulating layer 9a and 9b that is a layer of dielectric material, polymer, resist, glass, silicon oxide, etc. There are windows called contact windows 10a and 10b formed in the insulating layer/layers and the alignment electrode/electrodes make electric contact with the interior of the common channel via the contact window being electrically separated from the interior common channel over the remainder of their surface. The area of the electrode formed on the wall of the channel spreads from points A to B and from points C to D in Figures 11 a and 11 b. We shall call this area the mechanical contact area. So we can say that in these embodiments the contact window is a subset of the mechanical contact area. The benefit of these embodiments is that one could focus the alignment current over the sample fluid flow, reducing the overall current through the channel and concentrating it just at the desired location. Clearly one could construct various embodiments where one of the alignment electrodes is formed as described in the previous embodiments, the other one is formed with the contact window as in Figures 11a, 11b, 11c. Various other combinations of the alignment electrode configurations are possible. The contact window could be readily formed using the modern manufacturing methods of injection moulding, hot embossing, polymer printing or by using lithography methods that are ideally suited for forming window and openings in dielectric layers such as e.g. layers of resist SU8. Since the common channels and the alignment and detection electrodes are typically made using the lithography methods, the embodiments described in Figures 11a, 11b and 11c, can be readily fabricated. In the embodiment of Figure 11a, and Figure 11c the sample fluid is focused at the left upper corner of the common channel and along the upper wall of the channel respectively.

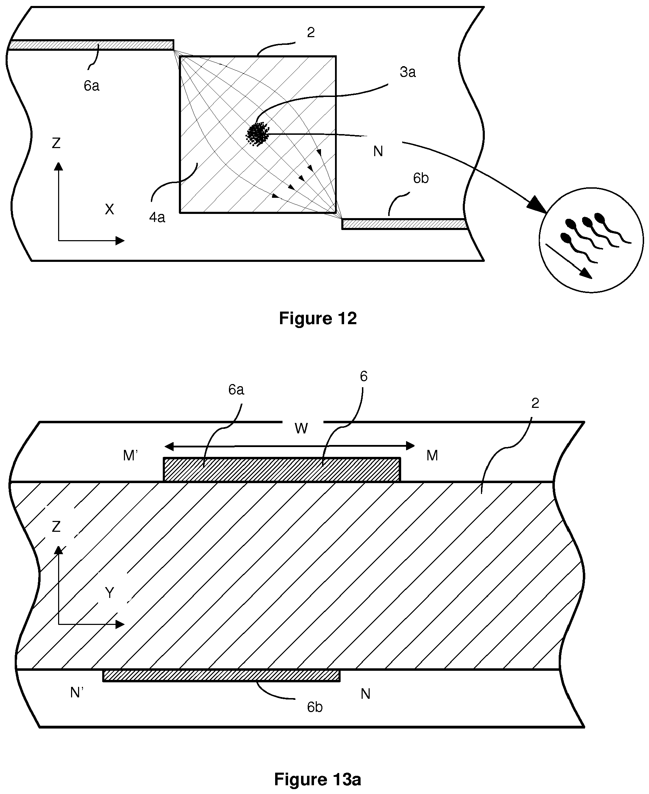

Figure 12 shows embodiment where the alignment electrodes are electrically insulated from the interior of the common channel. In other respects this embodiment is similar to the one presented in Figure 3a. Similarly embodiments with such alignment electrodes that are electrically insulated from the interior of the common channel, could be constructed based on other embodiments described in Figures 2-11. These will not be described here for brevity. Such an embodiment can be particularly beneficial when working with fluids that are electrically isolating as the field will not screened by the fluid and will penetrate the channel unimpeded. However, this could also be used even with fluids that are not electrically insulated. In this case it is preferable to active the alignment electrodes by an AC field. One could also construct embodiments where some of the alignment electrodes are electrically isolated from the interior of the common channel and others are not isolated as shown in the embodiments of Figures 2-11.

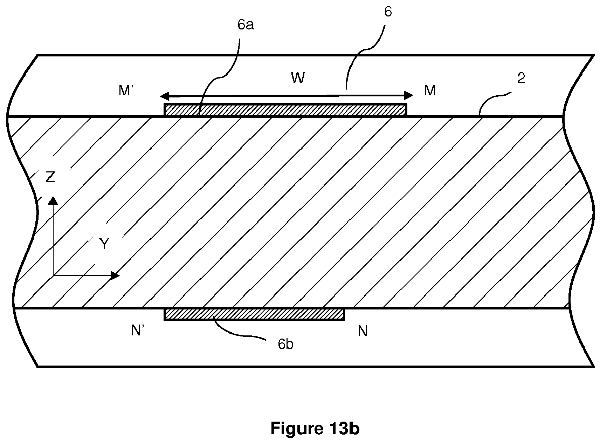

Figures 13a and 13b show two embodiments of the cross section of the common channel by a vertical plane along the flow direction. The directions of the z and y axes on the drawings are consistent with the X, Y, Z axes directions on the Figures 2-12. In these embodiments the edges of the alignment electrodes are marked by the letters M, N, M', N'. The lines MN are tilted with respect to the flow direction, i.e. the line MN is not parallel to the Y axis. In embodiment of Figure 13a, the line M'N' is also not parallel to the Y axis. This arrangement could be achieved by displacing one of the alignment electrodes with respect to the other one along the axis Y, parallel to the flow direction as shown on Figure 13a. This could also be achieved by making the two alignment electrodes of a different width W. The width is marked on Figure 13a to explain the notations. The benefit of this embodiment is that the field along the line MN of Figures 13a and 13b and along the line M'N' of Figure 13a is not orthogonal to the flow direction. In the areas deeper inside from the edges of the electrodes, the field is still orthogonal to the flow direction. Therefore this embodiment has benefits similar to the ones of Figure 7 of a continuously varying field direction along the length of the common channel.

## Claims

1. A microfluidic chip for microfluidic flow cytometry analysis of an anisotropic particulate containing fluid, the microfluidic chip comprising:
a microfluidic channel having a fluid inlet for receipt of a stream of anisotropic particulate containing fluid;
a detection zone comprising at least one pair of detection electrodes in electrical communication with the microfluidic channel configured to generate an AC detection electrical field between the detection electrodes and detect AC impedance changes in the microfluidic channel resulting from anisotropic particles passing between the detection electrodes; and
an alignment zone proximal to the detection zone located upstream from the detection zone comprising at least one pair of alignment electrodes in electrical communication with the microfluidic channel configured to generate an alignment electric field between the alignment electrodes.

2. A microfluidic chip according to Claim 1 in which the detection electric field and the alignment field are non-aligned.

3. A microfluidic chip according to Claim 1 or 2 in which the at least one pair of alignment electrodes is configured to generate the alignment electrical field across the microfluidic channel at an angle of 5-90° to the detection electrical field.

4. A microfluidic chip according to any preceding Claim in which the pair of alignment electrodes comprises an upper electrode disposed above and towards one side of the microfluidic channel and a lower electrode disposed below and towards an opposite side of the microfluidic channel.

5. A microfluidic chip according to any preceding Claim in which the alignment electrodes extend longitudinally along the microfluidic channel such that a degree of overlap between the alignment electrodes and the microfluidic channel varies longitudinally along the microfluidic channel.

6. A microfluidic chip according to any preceding Claim in which the alignment zone comprises one pair of alignment electrodes configured to align the anisotropic particulates at a first aligned position and a second pair of alignment electrodes distal of the first pair of alignment electrodes configured to rotate the anisotropic particulates to the second aligned position

7. A microfluidic chip according to any preceding Claim including hydrodynamic focussing means comprising a guidance microfluidic channel and a sample microfluidic channel that merge proximally of the alignment zone to inject the stream of sample fluid and the guidance fluid into the common microfluidic channel.

8. A microfluidic chip according to any preceding Claim, where the direction of the alignment electrical field/fields is chosen such as to be aligned preferably parallel or perpendicular with the anisotropic direction of the particulates resulting from the hydrodynamic focussing defined by the shape of the sample channel, guidance channel, the microfluidic channel and the configuration in which the sample channel and the guidance channel merge to form the microfluidic channel and the anisotropic shape of the particulates.

9. A microfluidic chip according to any preceding Claim, where the direction of the alignment electrical field is chosen such as to reduce the spread in angular alignment of the particulates present at the point of entry into the alignment zone.

10. A microfluidic chip according to any preceding Claim, wherein:
the at least one pair of alignment electrodes are configured to generate an AC electric field having a frequency such that complex dielectric permittivity of the particulates is significantly different from the one of the fluid carrying these particles; and/or
the alignment electrode/electrodes are positioned along the wall of the microfluidic channel over a mechanical contact area and yet are separated from the interior of the channel for a fraction of the mechanical contact area by a layer of dielectric and make electric contact with the interior of the channel over a fraction of the mechanical contact area via one or several windows formed in the layer of dielectric.

11. An apparatus comprising:
a microfluidic chip according to any of Claims 1 to 10;
at least one voltage and/or current generator electrically coupled to the pair of alignment electrodes; and
at least one voltage and/or current generator electrically coupled to the pair of detection electrodes

12. An apparatus according in Claim 11, in which the microfluidic chip comprises at least two pairs of alignment electrodes, each energised by a generator operating at different frequencies or one of the generators operates at DC voltage and the other one at AC voltage.

13. A method of microfluidic flow cytometry analysis of an anisotropic particulate containing fluid, the method comprising the steps of:
pumping an anisotropic particulate containing fluid through a microfluidic channel of a microfluidic chip according to any of Claims 1 to 10;
energising the at least one pair of alignment electrodes to align the particulates in the fluid in the alignment zone;
and detecting impedance changes in the detection zone caused by aligned particulates passing through the detection electrical field.

14. A method according to Claim 13 in which the at least one pair of alignment electrodes is configured to rotate the particulates into a first common alignment and then further rotate the particulates into a second common alignment.

15. A method according to any of Claims 13 to 14, in which the anisotropic particulates are cells, and in which the method is a method of separating cells according to phenotype or in which the anisotropic particulates are sperm cells, and in which the method is a method of separating sperm cells according to sex.
